# EUROPÄISCHE PATENTSCHRIFT

(11) **EP 1 404 719 B1**
(45) Veröffentlichungstag und Bekanntmachung des Hinweises auf die Patenterteilung: **27.02.2019**
(21) Anmeldenummer: 02735385.3
(22) Anmeldetag: 27.05.2002
(51) Int. Cl.: C08B 37/00, C08B 15/00, C08B 31/00, C08J 3/24, A61L 15/60, A61K 47/36, A61K 47/38

(54) **SUPERABSORBER, VERFAHREN ZU IHRER HERSTELLUNG UND IHRE VERWENDUNG**
SUPERABSORBER, METHOD FOR THEIR PRODUCTION AND USE THEREOF
SUPERABSORBANTS, LEUR PROCÉDÉ DE PRODUCTION ET LEUR UTILISATION

(30) Priorität: 25.05.2001 DE 10125599
(43) Veröffentlichungstag der Anmeldung: 07.04.2004
(73) Patentinhaber: Evonik Degussa GmbH, 45128 Essen (DE)
(72) Erfinder: MERTENS, Richard, 47803 Krefeld (DE); HÖLLER, Olaf, Greensboro, NC 27407 (US)
(74) Vertreter: Evonik Patent Association
(86) Internationale Anmeldenummer: PCT/EP2002/005799
(87) Internationale Veröffentlichungsnummer: WO 2002/096953

(56) Entgegenhaltungen:
- EP-A- 0 538 904
- EP-A- 0 637 594
- WO-A-95/11925
- DE-A- 19 807 504
- US-A- 3 589 364
- US-A- 4 952 550
- US-A- 5 252 340
- DATABASE WPI Week 199205 Derwent Publications Ltd., London, GB; AN 1992-038629 XP002214234 "Preparation of highly water-absorptive resin - by crosslinking polymer particles contg. carboxylic moiety and/or carboxylate moiety with crosslinking agent contg. reactive functional gps." & JP 03 285919 A (SEKISUI PLASTICS CO LTD) , 17. Dezember 1991 (1991-12-17)
- DATABASE WPI Week 199723 Derwent Publications Ltd., London, GB; AN 1997-256119 XP002214235 "Preparation of water absorptive cellulose material for sanitary or agricultural materials - comprises evaporating part of water from cellulose contg. material impregnated with aq. liq. contg. carboxylating agent etc. the heating with organic solvent contg. crosslinker." & JP 09 087956 A (OJI PAPER CO), 31. März 1997 (1997-03-31)
- DATABASE WPI Week 198813 Derwent Publications Ltd., London, GB; AN 1988-087257 XP002214236 "Highly water absorbing composn. - comprising mixt. of carboxymethyl cellulose and polyvalent metal salt" & JP 63 037143 A (DAICEL CHEM IND LTD), 17. Februar 1988 (1988-02-17)
- DATABASE WPI Week 200155 Derwent Publications Ltd., London, GB; AN 2001-499072 XP002214237 "Water absorbent for sanitary goods, engineering works and as coagulant, consists of crosslinked unit obtained by crosslinking carboxymethyl hydroxy-propyl galactomannan with titanium" & JP 2001 114803 A (UNITIKA LTD), 24. April 2001 (2001-04-24)
- "Investigations on drug release systems using CMC crosslinked with ferric ions" ART. CELLS, BLOOD SUBS., AND IMMOB. BIOTECH., Bd. 27, Nr. 3, 1999, Seiten 279-290, XP008008755

## Beschreibung

Die Erfindung betrifft Superabsorber auf Basis oberflächenmodifizierter Polycarboxypolysaccharide. Die erfindungsgemäßen Absorber besitzen eine hohe Aufnahmekapazität- und Geschwindigkeit, auch unter Druck, für Wasser und wässrige Lösungen, zeigen keine Neigung zum Gelblocking und sind mechanisch stabil. Die Absorptionsmaterialien sind alterungsstabil, toxikologisch unbedenklich und biologisch abbaubar. Die Erfindung betrifft ferner ein einfaches Verfahren zu ihrer Herstellung. Des Weiteren wird ihre Verwendung zur Absorption von Wasser, wässrigen Lösungen, Dispersionen und Körperflüssigkeiten in Hygiene- und Tierhygieneartikeln, in Verpackungsmaterialien für Lebensmittel, in Kulturgefäßen sowie zur Bodenverbesserung und als Kabelummantelung offenbart.

Die meisten der heute verwendeten Absorptionsmaterialien, die in der Lage sind, in kurzer Zeit große Flüssigkeitsmengen (Wasser, Urin) aufzunehmen, stellen in erster Linie schwach vernetzte, synthetische Polymere dar. Dazu zählen beispielsweise Polymere und Copolymere auf Basis von Acrylsäure oder Acrylamid, die nicht auf nachwachsenden Rohstoffen basieren und unzureichend bzw. überhaupt nicht biologisch abbaubar sind.

Während bei der Entwicklung der Superabsorber zunächst allein das sehr hohe Quellvermögen bei Kontakt mit Flüssigkeit, auch Absorption oder Free Swelling Capacity (FSC) genannt, allein im Vordergrund stand, hat sich später gezeigt, dass es nicht nur auf die Menge der absorbierten Flüssigkeit ankommt, sondern auch auf die Gelfestigkeit. Absorptionsvermögen oder auch FSC einerseits und Gelfestigkeit bei einem vernetzten Polymer andererseits stellten jedoch gegenläufige Eigenschaften dar, wie bereits aus dem US-PS 3,247,171 bekannt ist, ferner aus der US-PS Re 32 649. Das bedeutet, dass Polymere mit besonders hohem Absorptionsvermögen nur eine geringe Festigkeit des gequollenen Gels aufweisen mit der Folge, dass das Gel unter einem angewendeten Druck (z.B. Körperdruck) deformierbar ist und die weitere Flüssigkeitsverteilung und -aufnähme verhindert. Nach der US-PS Re 32 649 soll daher ein ausgewogenes Verhältnis zwischen Absorptionsvermögen und Gelstärke angestrebt werden, damit bei der Verwendung derartiger Superabsorber in einer Windelkonstruktion Flüssigkeitsaufnahme, Flüssigkeitstransport, Trockenheit der Windel und der Haut gewährleistet sind. Dabei kommt es nicht nur darauf an, dass das Polymer Flüssigkeit unter nachfolgender Einwirkung eines Druckes zurückhalten kann, nachdem das Polymer frei quellen konnte, auch Retention genannt, sondern auch darauf, Flüssigkeiten auch gegen einen gleichzeitigen, d.h. während der Flüssigkeitsabsorption, ausgeübten Druck aufzunehmen, wie es unter praktischen Gesichtspunkten geschieht, wenn ein Baby oder eine Person auf einem Sanitärartikel sitzt oder liegt oder wenn es, z.B. durch Beinbewegung zur Entwicklung von Scherkräften kommt. Diese spezifische Absorptionseigenschaft wird in der Edana-Methode 442.1-99 als Aufnahme gegen Druck ("Absorbency Against Pressure") oder kurz, AAP bezeichnet. Der für einen Superabsorber angegebene AAP-Wert wird dabei maßgeblich von dem aufgewendeten Druck, z.B. 21 oder 50g pro cm² bzw. 0,3 oder 0,7 psi aber auch durch das, bei der Messung gewählte Verhältnis der Superabsorbereinwaage zur Fläche, z.B. 0,032g pro cm² sowie die Korngrößenverteilung des Superabsorbergranulates, bestimmt.

Die EP 0 538 904 B1 bzw. die US 5,247,072 beschreiben Superabsorber auf Basis von Carboxyalkylpolysacchariden. In den Verfahren wird das Carboxyalkylpolysaccharid in Wasser gelöst und durch Trocknung oder Fällung isoliert und anschließend thermisch durch die Reaktion der Hydroxylgruppen des Polysaccharidskeletts mit den sauren Carboxylgruppen über interne Esterbrücken vernetzt. Da diese Vernetzungsreaktion sehr empfindlich auf geringe Änderungen des pH-Wertes, der Temperatur oder der Reaktionsdauer reagiert, werden Absorber mit stark schwankenden Absorptionseigenschaften erhalten. Die Materialien zeichnen sich durch ein hohes Absorptionsvermögen unter Druck aus, welches jedoch bei Lagerung der Absorber innerhalb weniger Wochen auf einen Bruchteil des ursprünglichen Aufnahmevermögens abfällt.

In der US 5,550,189 werden Absorber auf Basis von Carboxyalkylpolysacchariden beschrieben, bei denen die Alterungsstabilität durch Zusatz von mehrfachfunktionellen Vernetzern wie z.B. Aluminiumsalze oder Citronensäure verbessert wird. Die Herstellung der Absorber erfolgt aus einer gemeinsamen, homogenen wässrigen Lösung von Carboxyalkylpolysaccharid und Vernetzer, in der die Komponenten in geringer Konzentration vorliegen und aus der sie gemeinsam isoliert und dann thermisch vernetzt werden. Die Synthese dieser Absorber erfordert einen hohen Energie- und Zeitaufwand, da die wässrigen Lösungen nur sehr niedrig konzentriert sind. Die Verbesserung der Alterungsstabilität entspricht bei der Mehrzahl der Ausführungsbeispiele nicht den praxisrelevanten Anforderungen.

Die EP 855 405 A1 behandelt das Problem der mangelnden Alterungsbeständigkeit des Absorptionsvermögens quellbarer Stärkemaleate und schlägt als Lösung eine Anlagerung von Mercaptoverbindungen an die Doppelbindung des Maleinsäuresubstituenten vor. Das Absorptionsverhalten der Produkt, insbesondere unter Druckbelastung, ist sehr gering.

In der US 4,952,550 wird die Herstellung eines auf Carboxymethylcellulose basierenden Absorbers beschrieben, wobei die Carboxymethylcellulose in Wasser oder org. Lösemittel mit mehrwertigen Metallsalzen und einer hydrophobierenden Komponente behandelt wird. Eine thermische Vernetzung erfolgt nicht. Gemäss Offenbarung wird das Gelblocking bei diesen Absorbern durch die hydrophobierende Komponente gemindert.

Die Rohstoffe zur Herstellung von Superäbsorbern auf Polysaccharidbasis sind häufig wasserlöslich und müssen in die wasserunlösliche Form überführt werden, um sie als Superabsorber für Hygieneanwendungen verwenden zu können. Zahlreiche bekannte Verfahren beinhalten die homogene Vernetzung des absorbierenden Materials, um die Wasserlöslichkeit des Absorbers herabzusetzen. Dies hat häufig den Nachteil, dass solche homogen vernetzten Absorber nicht mehr das gewünschte Absorptionsvermögen gegenüber Flüssigkeiten aufweisen, da die Quellung durch die Vernetzung der Polymerketten zu stark eingeschränkt wird.

Weiterhin behindert die homogene Vernetzung die biologische Abbaubarkeit des Absorbers, da die Zugänglichkeit für Mikroorganismen durch die eingeschränkte Quellung vermindert wird. Darüber hinaus wird der enzymatische Abbau durch die zusätzlich eingeführten Substituenten gehemmt [Mehltretter et al., Journal of the American Oil Chemists Society, 47(1970)Seite 522-524]. In Versuchen, diese nachteiligen Eigenschaften zu verbessern, wurden verschiedene Methoden der Oberflächenbehandlung vorgeschlagen.

In der US 5,811,531 wird die Herstellung eines Absorbers basierend auf Uronsäuregruppen enthaltenden Polysacchariden wie Xanthan beschrieben, wobei die Polysaccharide an der Oberfläche mit mehrfach funktionellen organischen Vernetzern umgesetzt werden. Gemäss Offenbarung zeigen die Produkte ein besseres Absorptionsvermögen unter den Bedingungen des freien Quellens gegenüber salzhaltigen Lösungen als carboxyalkylierte Polysaccharide, bei denen die Carboxylgruppen nicht direkt, sondern über Alkylgruppen an die Saccharideinheiten gebunden sind

US 5,470,964 beschreibt die Herstellung eines an der Oberfläche mit mehrwertigen Metallionen vernetzten Absorbers auf Basis säuregruppenhaltiger Polysaccharide, der eine verbesserte Absorption gegen Druck aufweist. Nachteilig bei diesem Verfahren ist, dass für die verbesserte Aufnahmefähigkeit des Absorbers gegen Druck eine relativ dicke Schicht der Oberfläche vernetzt werden muss und dass dies gemäss Offenbarung nur durch vorheriges Anquellen des Polysaccharids mit großer Lösungsmittelmenge möglich ist. Im angequollenen Zustand können dann die mehrwertigen Metallionen tief genug in die Oberfläche eindringen. Um dies zu erreichen wird das Polysaccharid in einen Überschuss der wässrigen Metallsalzlösung gegeben wobei der Wasserüberschuss in Bezug auf das Polysaccharid bei der 2fachen bis 40fachen Menge liegt. Durch die dicke vernetzte Oberflächenschicht werden zwar gute Absorptionswerte gegen Druck erzielt, das freie Quellvermögen sowie das Retentionsvermögen des Absorbers wird dadurch jedoch nachteilig reduziert. Ferner ist bei dem beschriebenen Verfahren nachteilig, dass dem im Herstellungsprozess zuletzt in die Vernetzerlösung zugegebenen Teil des Polysaccharids weniger Quellzeit und eine geringere Vernetzerkonzentration zur Verfügung steht, so dass eine inhomogene Verteilung des Vernetzers auf der Oberfläche resultiert wodurch sich starke Schwankungen der Absorptionseigenschaften ergeben.
Der US 4,043,952 ist die Oberflächenbehandlung von wasserquellbaren, anionischen Polyelektrolyten mit polyvalenten Metallionen in einem für das Polymer unlöslichen Dispersionsmedium zu entnehmen, wodurch die Dispergierbarkeit der wasserabsorbierenden Produkte verbessert wird.
WO 95/11925 A1 beschreibt ein wasserabsorbierendes Polysaccharid, welches mit Zitronensäure oder mit einem Al³⁺ Salz vernetzt wird.
Allgemein lag der Erfindung die Aufgabe zu Grunde, die sich aus dem Stand der Technik ergebenden Nachteile zu überwinden.
Eine Aufgabe der vorliegenden Erfindung war es daher, biologisch abbaubare, superabsorbierende Polymere auf Basis nachwachsender Rohstoffe bereitzustellen, die die zuvor beschriebenen Mängel nicht aufweisen. Insbesondere sollen die Absorber eine hohe Langzeitlagerstabilität aufweisen, in der die Absorptionseigenschaften möglichst weitgehend erhalten bleiben. Gleichzeitig ist anzustreben, dass die Absorberpartikel eine hohe mechanische Stabilität aufweisen, um die Bildung von Feinstaubanteilen bei Verarbeitungsprozessen wie beispielsweise dem Sieben oder der Förderung zu vermeiden. Ferner sollten die Absorber hinsichtlich des Absorptionsverhaltens nicht zum Gelblocking neigen und neben einer hohen Absorptions- und Retentionskapazität auch eine hohe Aufnahmekapazität gegen Druck für Wasser und wässrige Lösungen besitzen. Für ein gutes Absorptions- und Anwendungsverhalten ist es erforderlich, dass die Absorber auch in einem Überschuss an wässriger Lösung einen überwiegend unlöslichen Charakter aufweisen.

Eine weitere Aufgabe der Erfindung ist es, ein Herstellungsverfahren für solche superabsorbierenden Polymere zu finden, das einfach, ökonomisch und sicher durchführbar ist, eine gleichmäßige Produktqualität liefert und bei dem niedrige Lösungsmittelmengen verwendet und organische Lösungsmittel nach Möglichkeit vermieden werden. Darüber hinaus sollen die Verfahren ohne die Verwendung toxikologisch bedenklicher Substanzen durchführbar sein.

Zudem besteht eine erfindungsgemäße Aufgabe darin, die biologische Abbaubarkeit von Hygieneartikeln wir Damenbinden, Wundauflagen, Inkontinenzartikeln und Windeln zu verbessern.

Die erfindungsgemäßen Aufgaben werden gelöst durch ein pulverförmiges, an der Oberfläche nachvernetztes Wasser, wässrige oder seröse Flüssigkeiten sowie Blut absorbierendes Polymerisat, erhältlich durch eine Oberflächenvernetzung mindestens eines teilneutralisierten, Carboxylgruppen enthaltenden Polysaccharids, nach Anspruch 1.

Erfindungsgemäß werden als Polysaccharidkomponente die Polycarboxypolysaccharide eingesetzt. Sie leiten sich entweder von Polysacchariden ab, die von Natur aus keine Carboxylgruppen enthalten und durch nachträgliche Modifizierung mit Carboxylgruppen versehen werden oder sie enthalten von Natur aus bereits Carboxylgruppen und werden gegebenenfalls nachträglich durch Modifizierung mit weiteren Carboxylgruppen versehen. Zur ersten Gruppe von Polysacchariden zählen beispielsweise Stärke, Amylose, Amylopektin, Cellulose und Polygalaktomannane wie Guar und Johannesbrotkernmehl, zur zweiten Gruppe zählen beispielsweise, Xanthan, Alginate, Gummi Arabicum.

Die Carboxylgruppen sind, wie bereits erwähnt, entweder durch den von Natur aus gegebenen Molekülaufbau vorhanden, beispielsweise durch Uronsäureeinheiten im Polysaccharidmolekül oder werden durch nachträgliche Modifizierung mit carboxylgruppenhaltigen Reagenzien eingebaut bzw. durch Oxidationsreaktionen erzeugt. Unter den Polycarboxypolysacchariden, bei denen die Carboxylgruppen durch nachträgliche Modifizierung eingebaut werden, sind die Carboxylalkylderivate bevorzugt, insbesondere die Carboxymethylderivate. Unter den Polycarboxypolysacchariden, bei denen die Carboxylgruppen durch Oxidation des Polysaccharidmoleküls erzeugt werden, sind insbesondere oxidierte Stärken und deren Derivate bevorzugt.

Die erfindungsgemäß zu verwendenden Polycarboxypolysaccharide sind wasserlöslich bzw. wasserquellbar und werden in nicht vernetzter Form eingesetzt.

Die erfindungsgemäß zu verwendenden Polycarboxypolysaccharide können neben den Carboxylgruppen mit weiteren Gruppen modifiziert sein, insbesondere solchen, die die Wasserlöslichkeit verbessern, beispielsweise Hydroxyalkyl-, insbesondere Hydroxyethylgruppen, sowie Phosphatgruppen.

Besonders bevorzugte Polycarboxypolysaccharide sind Carboxymethylguar, carboxylierte Hydroxyethyl- oder Hydroxypropylcellulose, Carboxymethylcellulose und Carboxymethylstärke, oxidierte Stärke, carboxylierte Phosphatstärke, Xanthan und Mischungen aus den einzelnen Polycarboxypolysacchariden. Insbesondere bevorzugt ist Carboxymethylcellulose.

Grundsätzlich sind erfindungsgemäß Polycarboxypolysaccharidderivate mit niedrigen und hohen Carboxyl-Substitutionsgraden einsetzbar. In einer bevorzugten Ausführungsform weisen sie einen durchschnittlichen Carboxyl-Substitutionsgrad im Bereich von 0,3 bis 1,5 auf, insbesondere bevorzugt werden Polycarboxypolysaccharidderivate mit einem Substitutionsgrad im Bereich von 0,4 bis 1,2.

Die bevorzugten wasserlöslichen Polycarboxypolyaccharidderivate haben im Rahmen der von dem natürlichen Polymeraufbau vorgegebenen Molekulargewichtsverteilung ein hohes mittleres Molekulargewicht und damit auch eine hohe Lösungsviskosität in verdünnter wässriger Lösung wie z.B. aus Baumwoll-Linters hergestellte Carboxymethylcellulose. Im Falle der Carboxymethylcellulose sind Derivate mit einer Lösungsviskosität in 1%iger wässriger Lösung von mehr als 2.000 mPas geeignet. Bevorzugt wird Carboxymethylcellulose mit einer Lösungsviskosität in 1%iger wässriger Lösung von mehr als 5.000 mPas eingesetzt und besonders bevorzugt von mehr als 7000 mPas.

Bedingt durch den Herstellungsprozess können Polycarboxypolysaccharide als Nebenbestandteil unterschiedlich hohe Salzmengen enthalten. Typische Salzgehalte von Carboxymethylcellulosen in Lebensmittelqualitäten liegen bei etwa 0,5 Gew.-%, bei technischen Qualitäten im Bereich von etwa 2 Gew.-% bis hin zu 25 bis 50 Gew.-% für Produkte in der Anwendung als Schutzkolloide. Obwohl die erfindungsgemäßen Absorber eine hohe Toleranz gegenüber einer Salzfracht aufweisen, sollten die zu verwendenden Polycarboxypolysaccharide nicht mehr als 15 Gew.-%, bevorzugt nicht mehr als 5 Gew.-% und besonders bevorzugt nicht mehr als 2 Gew. % Salz enthalten.

Zur Modifizierung der Absorber kann ein Zusatz carboxylgruppenfreier Polysaccharide erfolgen. Bevorzugt werden stark quellende Polysaccharide, wie z.B. Polygalaktomannane oder Hydroxyalkylcellulosen verwendet. Die zur Modifizierung einzusetzenden Mengen carboxylgruppenfreier Polysaccharide werden durch das geforderte Eigenschaftsprofil bestimmt, bevorzugt werden 20 Gew. %, vorzugsweise 10 Gew. % und besonders bevorzugt 5 Gew. % bezogen auf Polycarboxypolysaccharid verwendet.
Die Carboxylgruppen der Polycarboxypolysaccharide sind mindestens zu 80% bevorzugt zu mindestens 90% und ganz besonders bevorzugt zu 100% neutralisiert. Als Neutralisationsmittel haben sich Alkalihydroxide wie Natrium- und Kaliumhydroxid, Natrium- und Kaliumcarbonate bzw. Hydrogencarbonate und Ammoniumhydroxid und Amine bewährt.
Die physikalische Form der verwendeten Polysaccharidderivate ist für die Eigenschaften der erfindungsgemäßen Absorber ohne Bedeutung. Daher können die Polysaccharidderivate z.B. in Form von Pulvern, Feinstpulvern, Granulaten, Fasern, Flakes, Perlen oder Kompaktaten verwendet werden, wobei die Verwendung von pulverförmigen Materialien mit einer Korngröße im Bereich von 1 bis 2.000 µm aufgrund der einfachen Dosier- und Förderbarkeit bevorzugt wird.

Bei der Vorquellung des Polycarboxypolysaccharids in wässriger Phase können, bezogen auf das Polycarboxypolysaccharid, 0,01 bis 20 Gew.-%, bevorzugt 0,1 bis 10 Gew.-%, wasserlösliche Hilfsmittel und 0,01 bis 20 Gew.-%, bevorzugt 0,1 bis 10 Gew.-% Antiblockingadditive zugesetzt werden, die die Verarbeitbarkeit des entstehenden Hydrogels verbessern und die zumindest teilweise nach der Trocknung in dem Produkt verbleiben.

Wasserlösliche Hilfsmittel sind im Sinne der Erfindung aus der Gruppe der Basen, Salze und Treibmittel auszuwählen. Als Treibmittel werden anorganische oder organische Verbindungen gewählt, die unter Einfluss von Katalysatoren oder Wärme Gas freisetzen, beispielsweise Azo- und Diazoverbindungen, Carbonatsalze, Ammoniumsalze oder Harnstoff.

Weitere Hilfsmittel sind pH-Regulatoren wie z.B. Alkalimetallhydroxide, Ammoniak, basische Salze wie z.B. Alkalimetallcarbonate oder -Acetate. Weitere Hilfsmittel sind Neutralsalze, wie z.B. Alkalimetall- oder Erdalkalimetallsulfate oder -chloride zur Regulierung der Ionenstärke der Lösung bzw. des Salzgehaltes des pulverförmigen Absorberharzes.

Ferner können in dem wässrigen Hydrogel wassermischbare, organische Lösemittel, bevorzugt unter 100°C siedend, eingesetzt werden. Im Zuge der nachfolgenden Trocknung entweichen diese flüchtigen organischen Lösemittel weitestgehend aus dem Hydrogel. Bei der anschließenden Oberflächennachvernetzung werden diese Lösemittel dann endgültig verflüchtigt.

Die Antiblockingadditive, die das Gelblockingverhalten des pulverförmigen Absorberharzes weiter reduzieren sind z.B. native- oder synthetische Fasermaterialien oder andere Materialien mit einer großen Oberfläche z.B. aus der Gruppe der Kieselgele und synthetischen Kieselsäuren und der wasserunlöslichen Mineralsalze.

Die erfindungsgemäßen Absorber sind an der Oberfläche nachvernetzt. Im Anschluss an die thermische Trocknung, Zerkleinerung und Klassierung des Hydrogels erfolgt diese Vernetzung der Oberfläche der Polycarboxypolysaccharidpulver mit kovalenten und/oder ionischen Vernetzern, die mit den oberflächennahen funktionellen Molekülgruppen, vorzugsweise Carboxyl-, Carboxylat- oder Hydroxylgruppen, vorzugsweise unter Erhitzung reagieren. Oberflächenvernetzer werden in einer Menge von 0,01 - 25 Gew.-%, vorzugsweise 0,1 - 20 Gew.-% bezogen auf das Polysaccharid eingesetzt.
Als kovalente Oberflächennachvernetzungsmittel, die auch in Kombination mit ionischen Vernetzern eingesetzt werden können, werden solche Vernetzer eingesetzt, die mit den funktionellen Gruppen der Polycarboxypolysaccharide unter Ausbildung kovalenter Bindungen reagieren. In einer bevorzugten Ausführungsform werden Vernetzer eingesetzt, die mit den Hydroxylgruppen des Absorberharzes reagieren können, beispielsweise Säuregruppen enthaltende Substanzen. Insbesondere sind niedermolekulare Polycarbonsäuren und deren Derivate wie z.B. Malonsäure, Maleinsäure, Maleinsäureanhydrid, Weinsäure und polymere Polycarbonsäuren, z.B. auf Basis von (Meth)Acrylsäure und oder Maleinsäure geeignet. Bevorzugt werden Citronensäure, Butantetracarbonsäure und Polyacrylsäure, besonders bevorzugt wird die Citronensäure verwendet. Citronensäure wird bevorzugt in einer Menge von 0,2 - 8 Gew.-%, insbesondere bevorzugt 0,3 - 6 Gew.-% bezogen auf das Polycarboxypolysaccharid eingesetzt. Die Polycarbonsäuren können auch in teilneutralisierter Form, z.B. durch teilweise Neutralisation mit Alkalihydroxiden oder Aminbasen verwendet werden.

Geeignete ionische Nachvernetzungsmittel, die alleine oder in Kombination mit den kovalenten Nachvernetzungsmitteln verwendet werden können sind Salze Al³⁺. Aluminiumsalze werden bevorzugt in einer Menge von 0,2 - 1,0 Gew.-%, vorzugsweise 0,25 - 0,85 Gew.-% bezogen auf das Polycarboxypolysaccharid eingesetzt.

Weitere geeignete Nachvernetzungsmittel sind solche, die sowohl kovalente als auch ionische Vernetzungsbindungen eingehen können, z.B. Di- und Polyamine die sowohl als kovalente Vernetzer, über Amidgruppen, wie auch als ionische Vernetzer, über Ammoniumsalzkomplexe, fungieren können.
Die kovalente Oberflächennachvernetzung kann gegebenenfalls durch Katalysatoren beschleunigt werden. Als Katalysatoren werden bevorzugt Verbindungen eingesetzt, die die Veresterungsreaktion zwischen einer Carboxylgruppe und einer Hydroxylgruppe katalysieren wie z.B. Hypophosphite, Acetylacetonate, Mineralsäuren, wie z.B. Schwefelsäure und Lewis Säuren. Bevorzugt werden Schwefelsäure und Hypophosphit verwendet. Das Gewichtsverhältnis von Oberflächennachvernetzer zu Vemetzungskatalysator beträgt 1:0,001 - 1:1, vorzugsweise 1:0,1-2:1.
Optional kann die Lösung mit der der Oberflächennachvernetzer auf das Polycarboxypolysaccharid aufgetragen wird ein oder mehrere wasserlösliche Hilfsmittel enthalten, die die homogene Verteilung der Vernetzerlösung auf der Oberfläche der Absorber fördern. In einer bevorzugten Ausführungsform enthält die Lösung bis zu 40 Gew.-% dieser Hilfsmittel. Derartige Hilfsmittel sind neben wassermischbaren organischen Lösungsmitteln wie beispielsweise Ethanol, Propanol, 2-Propanol, Aceton, Glycerin, Tetrahydrofuran und Dioxan auch wasserlösliche organische Feststoffe wie z.B. Polyalkylenglykole, Polyvinylalkohole und Polyacrylsäuren. Von den organischen Feststoffen wird bevorzugt Polyethylenglykol verwendet. Der bevorzugte Molekulargewichtsbereich des Polyethylenglykols liegt bei größer gleich 1000, insbesondere bei größer gleich 1500.

In einer bevorzugten Ausführungsform fungieren die AluminiumSalze gleichzeitig als ionischer Oberflächenvernetzer und als Hilfsmittel für die homogene Verteilung der Vernetzerlösung auf der Oberfläche.
Die erfindungsgemäßen teilchenförmigen Absorberharze zeigen ein sehr gutes Retentions- und Absorptionsvermögen und ein signifikant verbessertes Aufnahmevermögen für Wasser und wässrige Flüssigkeiten entgegen einem äußeren Druck in Kombination mit einer ausgezeichneten Alterungsstabilität.
Die hervorragende Alterungsstabilität zeigt sich darin, dass nach Lagerung der Produkte unter Normalbedingungen nach 200 Tagen immer noch mindestens 80% der Werte der Absorption unter Druck (AAP_{0,7}) vorhanden sind.

Die ausgezeichneten Absorptionseigenschaften der erfindungsgemäßen Absorber zeigen sich darin, dass sie mit einer Retention von größer oder gleich 20 g/g bei einer Absorption gegen Druck (AAP_{0,7}) von mindestens 11g/g, bevorzugt von mindestens 15 g/g herstellbar sind und bevorzugt mit einer Retention von größer oder gleich 25g/g bei einer Absorption gegen Druck (AAP_{0,7}) von mindestens 11 g/g, bevorzugt von mindestens 15 g/g.

Die Schüttdichte der erfindungsgemäßen teilchenförmigen Absorberharze bewegt sich im technisch üblichen Bereich und liegt für gewöhnlich unter 1000 g/dm³. In einer bevorzugten Ausführungsform liegt das Produkt mit einer Schüttdichte von kleiner 800 g/dm³ und besonders bevorzugt kleiner 600 g/dm³ vor.

Besonders hervorzuheben ist auch die überraschende Stabilität der erfindungsgemäßen Absorber gegen Abrieb. So werden unter den mahlenden Bedingungen in einer Kugelmühle innerhalb von 6 Minuten (siehe Prüfmethode "Mechanische Stabilität") weniger als 5% Feinkornanteil unterhalb von 150µm gebildet. Diese hohe Abriebstabilität ermöglicht eine weitgehend staubfreie Verarbeitung der Absorber z.B. in Produktionsanlagen zur Windelherstellung, in denen die Absorber bei der Produktförderung mechanischer Belastung ausgesetzt sind.
Hervorzuheben ist auch die biologische Abbaubarkeit unter Kompostbedingungen, bei der nach einem Zeitraum von 90 Tagen ein Abbau zu Wasser und Kohlendioxid von mindestens 40% vorliegt und kontinuierlich weiter abgebaut wird.
Die erfindungsgemäße Oberflächennachvernetzung konzentriert sich, im Gegensatz zu den Produkten des Standes der Technik, auf eine geringe äußere, äußerst stabile Schicht. Das wird durch die Messung des sogenannten Surface-Crosslinking-Index (SCI) ermittelt, der sich aus der Differenz der Vernetzerkonzentrationen im durch Abrieb ermittelten Feinstaub und dem nicht dem Abrieb unterworfenen Absorber ergibt. Je höher sich der SCI-Index einstellt, desto mehr Vernetzer wurde mit dem Feinstaub vom Absorber abgetragen, d.h. desto konzentrierter ist der Vernetzer auf der äußeren Schicht des Absorbers vorhanden. Erfindungsgemäße Absorber weisen einen SCI-Index von größer 40 auf. Bei Absorbern mit niedrigeren SCI-Werten ist der Oberflächenvernetzer tiefer in das Polymerteilchen eingedrungen, was zu einer Verminderung der Absorptionseigenschaften führt.

Im weiteren wird die erfindungsgemäße Aufgabe durch ein einfach, ökonomisch und sicher durchführbares Verfahren zur Herstellung der mechanisch stabilen, oberflächig nachvernetzten Polymerpartikel mit signifikant verbesserten Absorptionseigenschaften bei gleichbleibender Produktqualität gemäß Anspruch 9 gelöst, durch Vernetzen der Oberfläche eines Polycarboxypolysaccharids mit einem Oberflächenvernetzer, dadurch gekennzeichnet, dass aus einem unvernetzten Polycarboxypolysaccharid mit Wasser ein Hydrogel gebildet wird, das Hydrogel mechanisch zerkleinert und getrocknet wird, eine Zerkleinerung und Klassierung des getrockneten Hydrogels unter Ausbildung eines Polymerpulvers erfolgt und dass die Partikel des Polymerpulvers mit einer Lösung eines Vernetzers beschichtet und anschließend einer Oberflächennachvernetzung unterworfen werden.

Überraschenderweise führt das erfindungsgemäße Verfahren zu teilchenförmigen Absorberharzen mit sehr gutem Retentions- und Absorptionsvermögen und einem signifikanten verbesserten Aufnahmevermögen für Wasser und wässrige Flüssigkeiten entgegen einem äußeren Druck in Kombination mit einer ausgezeichneten Alterungsstabilität sowie einer deutlich reduzierten Löslichkeit in wässrigen Lösungen.

Völlig unerwartet liefert das erfindungsgemäße Verfahren alterungsstabile Superabsorberharze, die auch bei Lagerung über einen langen Zeitraum ihre sehr guten Absorptionseigenschaften beibehalten und gleichwohl unter Kompostbedingungen kontinuierlich biologisch abgebaut werden.

In der ersten Stufe des erfindungsgemäßen Verfahrens wird das Polycarboxypolysaccharidderivat zusammen mit einem Lösungsmittel in ein festes Hydrogel überführt, welches gegebenenfalls noch weitere Additive oder Hilfsmittel enthält. Als Lösungsmittel werden besonders bevorzugt Wasser sowie Mischungen aus Wasser mit organischen Lösungsmitteln wie z.B. Ethanol, Propanol, Butanol, 2-Propanol oder Aceton verwendet. In einer Ausführungsform wird das Polycarboxypolysaccharid in einem Gemisch aus Wasser und organischem Lösemittel, gegebenenfalls unter erhöhter Temperatur, vorsuspendiert und nach dem Abtrennen aus der Suspension in das Hydrogel überführt.

Die Herstellung des Hydrogels erfolgt bevorzugt durch mechanisches Mischen des Polycarboxypolysaccharidderivates mit der Lösungsmittelkomponente in einer kontinuierlichen oder diskontinuierlichen Arbeitsweise. Geeignete Mischeinrichtungen stellen z.B. diskontinuierliche Kneter wie Trogkneter, Innenmischer oder kontinuierliche Kneter wie Ein-, Zwei- oder Mehrwellenmischer dar.

Bei der Herstellung des Hydrogels kann der Gehalt an Polycarboxypolysaccharid in der Mischung aus Polycarboxypolysaccharid und Wasser in weiten Grenzen schwanken, in einer bevorzugten Ausführungsform des Verfahrens liegt er im Bereich von 5 bis 65 Gew. % und besonders bevorzugt 5 bis 55 Gew. %. Aus Gründen der besseren Verarbeitbarkeit des Hydrogels kann es mitunter erforderlich sein, dass der Gehalt an Polycarboxypolysaccharid 45 Gew. % nicht überschreitet.

In einer bevorzugten Ausführungsform wird das Lösemittel dem trockenen Rohstoff Polycarboxypolysaccharid kontinuierlich zugeführt, beispielsweise in einem Extruder, wobei das Verfahren so geführt wird, dass das Lösemittel im Unterschuss vorliegt.

Überraschenderweise wurde gefunden, dass die Absorptionseigenschaften der erfindungsgemäßen Superabsorber durch die Effektivität der Mischung bzw. die Homogenität des primär hergestellten Hydrogeles nur gering beeinflusst werden.

Die Mischung der Einzelkomponenten in einem kontinuierlich arbeitenden Mischreaktor führt z.B. mit steigendem Durchsatz zu weniger homogenen Hydrogelen mit zunehmenden Anteilen an trockenen, nicht gequollenen Polymeranteilen. Es wird angenommen, dass im Verlaufe der Weiterverarbeitung zu pulverförmigen Ab-sorberharzen eine Nachquellung stattfindet, so dass letztlich eine, im Vergleich zu vollständig homogen gemischten Gelen identische Absorptionsleistung resultiert.

Die Mischung aus Polycarboxypolysaccharid und Wasser kann erfindungsgemäß zusätzlich bis zu 30 Gew. %, vorzugsweise bis zu 20 Gew. % eines oder mehrerer, mit Wasser mischbarer und mit dem Polycarboxypolysaccharid nicht mischbarer organischer Lösemittel enthalten.

Das Verhältnis von Feststoffkomponenten zu Lösungsmittelkomponenten kann in weiten Grenzen variieren und wird bevorzugt so gewählt, dass das resultierende Hydrogel eine feste und wenig klebrige Konsistenz hat. Als besonders günstig hat sich erwiesen, wenn das gequollene Gel nach Förderung z.B. mit einem Fleischwolf oder einem Extruder und Formgebung mittels einer Lochscheibe in Form von festen Strängen vorliegt, die auch bei längerer Lagerung keine Tendenz zum gegenseitigen Verkleben aufweisen. Die Gelkonsistenz kann über den Gewichtsanteil organischer, wasserlöslicher Lösungsmittel im Hydrogel gezielt eingestellt werden. Je geringer dabei die Konzentration des Derivats des Polycarboxypolysaccharids im Hydrogel ist, desto höher muss der Gewichtsanteil des organischen Lösungsmittels gewählt werden, um die bevorzugte Gelkonsistenz zu erzielen. Wird z.B. als Derivat des Polycarboxypolysaccharids eine hochmolekulare Carboxymethylcellulose mit einer Lösungsviskosität in 1%iger wässriger Lösung von mehr als 4.000 mPas eingesetzt und als Lösungsmittel reines Wasser verwendet ergibt sich die bevorzugte Gelkonsistenz bei einem Polymergehalt von mehr als 15 Gew.-% bezogen auf das gequollene Gel. Reduziert man den Polymeranteil innerhalb des Gels auf weniger als 15 Gew.-% wird ein weiches und klebriges Gel erhalten, das nicht die bevorzugte Konsistenz aufweist. Werden jedoch 1-20 Gew.-%, vorzugsweise 5-15 Gew.-% des Lösungsmittels Wasser durch ein organisches, wassermischbares Lösungsmittel wie z.B. 2-Propanol ersetzt, welches ein Fällungsmittel für die Carboxymethylcellulose darstellt und eine schlechtere Löslichkeit des Polymers in dem Lösungsmittelgemisch bewirkt, zeigen auch Hydrogele mit einem Polymeranteil von weniger als 15 Gew.-% die bevorzugte Gelkonsistenz. Reduziert man den Polymeranteil auf weniger als 10 Gew.-% muss der Anteil des organischen Lösungsmittels entsprechend weiter auf mehr als 15% erhöht werden um die bevorzugte Gelkonsistenz zu erzielen.

Die Anwesenheit eines organischen, wasserlöslichen Lösungsmittels im gequollenen Gel wirkt sich nicht nur positiv auf die Gelkonsistenz aus, sondern verbessert überraschenderweise auch die Absorptionseigenschaften des pulverförmigen Superabsorbers signifikant. Dieser Effekt tritt auch schon bei geringen Mengen von weniger als 5 Gew.-% bezogen auf das Gel deutlich zutage und äußert sich bei dem Absorberharz vor allem in einer signifikant höheren Aufnahmekapazität für wässrige Flüssigkeiten gegen Druck.

Optional kann das Lösungsmittel bzw. das Lösungsmittelgemisch noch 0,01-20 Gew. % vorzugsweise 0,1-10 Gew. %, bezogen auf den Feststoffgehalt, eines oder mehrerer wasserlöslicher Hilfsmittel aus der Gruppe der Basen, Salze und Treibmittel enthalten, die die Verarbeitbarkeit des gequollenen Geles oder die Absorptionseigenschaften des Absorberharzes verbessern sowie eine Vernetzungsreaktion während des Trocknungsprozesses unterbinden. Bevorzugte Hilfsmittel sind zum einen pH-Regulatoren wie z.B. Alkalimetallhydroxide, Ammoniak, basische Salze wie z.B. Alkalimetallcarbonate oder -Acetate. Weitere bevorzugte Hilfsmittel sind Neutralsalze wie z.B. Alkalimetall- oder Erdalkalimetallsulfate oder - chloride zur Regulierung der Ionenstärke der Lösung bzw. des Salzgehaltes des pulverförmigen Absorberharzes. Als zusätzliche Hilfsmittel werden vorzugsweise Verbindungen eingesetzt, die unter der Einwirkung von Katalysatoren oder Wärme Gase freisetzen (Treibmittel) und somit dem Hydrogel bzw. dem Absorberharz zusätzliche Porosität verleihen, wodurch die Absorptionseigenschaften des Absorberharzes zusätzlich verbessert werden. Typisch zu verwendende Treibmittel sind z.B. Azo- und Diazoverbindungen, Carbonatsalze, Ammoniumsalze oder Harnstoff.

Gegebenenfalls kann das Hydrogel noch 0,01-20 Gew. %, vorzugsweise 0,1-10 Gew. % eines oder mehrerer Antiblockingadditive enthalten, die das Gelblockingverhalten des pulverförmigen Absorberharzes weiter reduzieren. Geeignete Antiblockingadditive sind z.B. native- oder synthetische Fasermaterialien oder andere Materialien mit einer großen Oberfläche z.B. aus der Gruppe der Kieselgele, synthetische Kieselsäuren und weitgehend wasserunlösliche Mineralsalze.

In der nächsten'Stufe des erfindungsgemäßen Verfahrens wird das Hydrogel zerkleinert und bis auf einen geringen Restwassergehalt getrocknet. Der Zerkleinerungs- und Trocknungsschritt kann sich dabei unmittelbar an die Vorquellung anschließen, es ist aber auch möglich, die Hydrogele vor der Weiterverarbeitung für einen längeren Zeitraum, z.B. mehrere Wochen zwischenzulagern, ohne dass sich die Eigenschaften der daraus resultierenden erfindungsgemäßen Superabsorber ändern. Durch die Gelzerkleinerung wird vor allem das Verhältnis von Geloberfläche zu Gelvolumen vergrößert, wodurch der nachfolgende Trocknungsschritt wesentlich weniger Energieeintrag benötigt. Das Verfahren der Gelzerkleinerung unterliegt keiner Einschränkung. In einer besonders bevorzugten Ausführungsweise erfolgt die Gelzerkleinerung durch die Verpressung des Gels durch eine Lochscheibe zu Gelsträngen, die gegebenenfalls durch ein Schneidwerkzeug in kürzere Gelstränge zerteilt werden können.

Zur Trocknung der Hydrogelteilchen sind verschiedene Verfahren bekannt. Mögliche Verfahren sind z.B. die Verdampfungstrocknung, Verdunstungstrocknung, Strahlungstrocknung (Beispiel: Infrarottrocknung), Hochfrequenztrocknung (Beisp.: Mikrowellentrocknung), Vakuumtrocknung, Gefriertrocknung oder Sprühtrocknung. So kann die Trocknung beispielsweise nach dem Dünnfilm-Trockenverfahren, z.B. mit Hilfe eines Zweiachsen-Walzentrockners, nach dem Plattentrockenverfahren, gemäß dem die Hydrogelpolymerteilchen auf Platten in mehreren Schichten in eine Trockenkammer geladen werden, in der Heißluft zirkuliert, nach dem Drehtrommel-Verfahren mit Hilfe von Walzentrocknern oder nach dem Förderband-Verfahren, im folgenden auch als Bandtrocknung bezeichnet erfolgen. Die Bandtrocknung, bei der mit Löcher versehene Horden eines Kreisförderers in einem Tunnel mit Trocknungsgut beladen und das Trocknungsgut während der Förderung durch Durchblasen von Heißluft durch die Hordenlöcher getrocknet wird, stellt das wirtschaftlichste Trocknungsverfahren für wasserquellbare hydrophile Hydrogele dar und ist daher bevorzugt.

Die Feuchtigkeit des durch Trocknung des Hydrogels entstandenen Polymerpulvers liegt vorteilhafter Weise nicht über 30 Gew. %, vorzugsweise nicht über 15 Gew. % und besonders bevorzugt nicht über 10 Gew. %.
Die Trocknung des Polymerisatgels erfolgt bei Temperaturen oberhalb von 70°C, bevorzugt oberhalb von 120°C und besonders bevorzugt oberhalb von 130°. Die Parameter wie der Polymergehalt des Hydrogeles, der pH-Wert des Lösungsmittelsystems, das Mischverfahren, die Trocknungstemperatur und die Trocknungsdauer beeinflussen sieh gegenseitig und werden bevorzugt so aufeinander abgestimmt, dass während der Trocknung keine interne Vernetzung des Hydrogels erfolgt. Wird z.B. bei der Herstellung des Hydrogeles ein Lösungsmittel mit einem pH-Wert unterhalb von 7 verwendet, wird ein Teil, der im Polysaccharidderivat vorhandenen Carboxylatgruppen in die freie Säureform überführt, welche vor allem gegen Ende der Trocknung durch eine Veresterung mit den Hydroxylgruppen als interne Vernetzer fungieren können. Um diese, im Prinzip unerwünschte, interne Vernetzung zu vermeiden oder weitgehend zurückzudrängen erfolgt die Trocknung in diesen Fällen bevorzugt bei Temperaturen im Bereich von 70-100°C. Der pH-Wert wird für gewöhnlich auf 6 oder höher eingestellt. In einer bevorzugten Ausführung der Erfindung wird für die Herstellung des Hydrogels ein Lösungsmittel mit einem pH-Wert von ≥ 7 gewählt und die Trocknung bei Temperaturen ab 120°C, vorzugsweise ab 130 bis 160°C durchgeführt.

Wird das Hydrogel in einem kontinuierlichen Mischer hergestellt, beispielsweise in einem Extruder, so können die noch nicht an der Oberfläche nachvernetzten Vorprodukte bereits ab pH-Werten von 7 hohe Retentionen von größer oder gleich 40 g/g aufweisen, die sich beim Tempern über 60 Minuten und 120°C als stabil erweisen und die sich von Produkten, die mit höheren pH-Werten hergestellt wurden, nur noch geringfügig unterscheiden. Werden die Hydrogele dagegen in einem Batch- Prozess hergestellt, so steigt die Stabilität gegenüber einer Temperung mit steigendem pH-Wert des Hydrogels an. Eine bevorzugte pH-Einstellung bei der Hydrogelbildung im Batch-Prozess liegt bei pH 10 oder höher.

Überraschenderweise wurde gefunden, dass bei den besonders bevorzugten Trocknungstemperaturen oberhalb von 130°C, die eine partielle Verhornung der Hydrogelteilchen bewirken, superabsorbierende Polymere mit signifikant höherem Absorptions- und Retentionsvermögen bei vergleichbarem Absorptionsvermögen gegen einen äußeren Druck erhältlich sind.

Für die nachfolgende Mahlung der getrockneten Hydrogelteilchen ist es vorteilhaft, das Trockengut im letzten Abschnitt der bevorzugten Bandtrocknung auf Temperaturen <70°C, bevorzugt <60°C und besonders bevorzugt <50°C abzukühlen. Die getrockneten, abgekühlten Hydrogelteilchen werden zunächst vorgebrochen, beispielsweise mit Hilfe eines Fingerbrechers. Die so vorzerkleinerten Hydrogelteilchen werden dann gemahlen, wobei die Mahlung bevorzugt mit Hilfe eines Walzenstuhls erfolgt, um den Anfall an Feinteilen möglichst gering zu halten. In einer besonders bevorzugten Ausführung erfolgt die Mahlung zweistufig, erst über einen Grobwalzenstuhl, dann über einen Feinwalzenstuhl, wobei letzterer wiederum ein- oder zweistufig sein kann.

Bei der anschließenden Siebung wird die Korngrößenverteilung eingestellt, die in der Regel zwischen 10 und 3000 µm, bevorzugt zwischen 100 und 2000 µm und besonders bevorzugt zwischen 150 und 850 µm liegt. Zu grobe Partikel können erneut der Mahlung unterworfen werden, zu feinteilige Partikel können dem Herstellungsprozess zurückgeführt werden.

Die Oberflächenbeschichtung des getrockneten pulverförmigen Polymerisates mit 0,01 bis 25 Gew. %, bevorzugt 0,1 bis 20 Gew. % bezogen auf das Polymerisat eines Nachvernetzers, der in Form einer 0,01 bis 80 Gew.-%, vorzugsweise 0,1 bis 60 Gew.-% Lösung zugeführt wird, erfolgt in geeigneten Mischaggregaten. Dies sind beispielsweise Paterson-Kelly-Mischer, DRAIS-Turbulenzmischer, Lödigemischer, Rubergmischer, Schneckenmischer, Tellermischer, Wirbelschichtmischer oder Schugi-Mischer. Nach Aufsprühen der Vernetzer-Lösung kann ein Temperaturbehandlungsschritt folgen, bevorzugt in einem nachgeschalteten Trockner, bei einer Temperatur zwischen 40 und 250°C, bevorzugt 60-200°C und besonders bevorzugt 80-160°C, über einen Zeitraum von 5 Minuten bis 6 Stunden, bevorzugt 10 Minuten bis 2 Stunden und besonders bevorzugt 10 Minuten bis 1 Stunde, wobei Lösungsmittelanteile entfernt werden. Die optimale Zeitdauer der Nacherhitzung kann für die einzelnen Vernetzertypen mit wenigen Versuchen leicht ermittelt werden. Sie wird dadurch begrenzt, wenn das gewünschte Eigenschaftsprofil des Superabsorbers infolge von Hitzeschädigung wieder zerstört wird. Die thermische Behandlung kann in üblichen Trocknern oder Öfen durchgeführt werden; beispielhaft seien Drehrohröfen, Wirbelbetttrockner, Tellertrockner, Paddeltrockner oder Infrarottrockner genannt.

Es hat sich teilweise als vorteilhaft erwiesen, dass die wässrige Lösung des Oberflächennachvernetzers vor ihrem Einsatz auf eine Temperatur von 15°C-100°C, vorzugsweise auf 20°C - 60°C eingestellt wird.

Die kovalente Oberflächennachvernetzung kann gegebenenfalls durch Katalysatoren beschleunigt werden. Als Katalysatoren werden bevorzugt Verbindungen eingesetzt, die die Veresterungsreaktion zwischen einer Carboxylgruppe und einer Hydroxylgruppe katalysieren wie z.B. Hypophosphite, Acetylacetonate, Mineralsäuren, wie z.B. Schwefelsäure und Lewis Säuren. Bevorzugt werden Schwefelsäure und Hypophosphit verwendet. Das Gewichtsverhältnis von Oberflächennachvernetzer zu Vernetzungskatalysator beträgt 1:0,001 - 1:1, vorzugsweise 1:0,1 - 2:1

In einer bevorzugten Ausführungsform werden die Vernetzungskatalysatoren der Lösung des Oberflächennachvernetzers zugemischt.

Optional kann die Nachvernetzungslösung bis zu 70 Gew.-% eines oder mehrerer Hilfsmittel enthalten. Hilfsmittel sind vor allem wasserlösliche Verbindungen, die die homogene Verteilung der Vernetzerlösung auf der Oberfläche der Absorber fördern, indem sie die Penetration des Lösungsmittels in das Innere der Superabsorberpartikel verlangsamen sowie die Löslichkeit der Partikeloberfläche und damit die Tendenz der feuchten Superabsorberpartikel miteinander zu verkleben reduzieren. Bevorzugte Hilfsmittel sind neben wassermischbaren organischen Lösungsmitteln wie beispielsweise Ethanol, Propanol, 2-Propanol, Aceton, Glycerin, Tetrahydrofuran und Dioxan auch wasserlösliche hydrophile organische Feststoffe, insbesondere Polymere wie z.B. Polyalkylenglykole, Polyvinylalkohole, bevorzugt Polyäthylenglykole.

In einer bevorzugten Ausführungsform fungieren die AluminiumSalze gleichzeitig als ionischer Oberflächenvernetzer und als Hilfsmittel für die homogene Verteilung der Vernetzerlösung auf der Oberfläche.
Die erfindungsgemäßen Polymere zeichnen sich durch ein hervorragendes Absorptions- und Retentionsvermögen für Wasser, wässrige Lösungen und Körperflüssigkeiten aus. Gleichzeitig verfügen die superabsorbierenden Polymere durch die gezielte Vernetzung der Oberfläche über ein deutlich verbessertes Absorptionsvermögen für wässrige Lösungen gegen einen äußeren Druck. Darüber hinaus sind die erfindungsgemäßen Superabsorber auf Basis von Polycarboxypolysaccharidderivaten lagerstabil, frei von Restmonomerenanteilen, nur in geringem Maße in wässrigen Flüssigkeiten löslich und biologisch abbaubar.
Die erfindungsgemäßen Superabsorber sind in hervorragender Weise als Absorptionsmittel in Hygieneartikeln wie z.B. Baby- und Erwachsenenwindeln, Wundabdeckungen, Damenbinden, Tampons und dergleichen geeignet. Insbesondere eignen sich die Polymere zum Einsatz in Hygieneartikeln, die nach Gebrauch einer Kompostierung zugeführt werden sollen, da sie sich in Kompostierungstests nach ASTM-Methode D 5338-92 vom 15.12.1992, nach CEN Entwurf "Evaluation of the Ultimate Aerobic Biodegradability and Disintegration of Packaging Materials under Controlled Composting Conditions" vom 6.5.1994 und nach DIN 54900, Teil 2, Verfahren 3 vom Januar 1997 als biologisch abbaubar erwiesen haben.
Absorbierende Hygieneprodukte besitzen in der Regel einen allgemeinen Aufbau aus einer körperzugewandten flüssigkeitsdurchlässigen Abdeckung (1), einer flüssigkeitsabsorbierenden Sauglage (2) sowie einer im wesentlichen flüssigkeitsundurchlässigen, körperabgewandten Außenschicht (3). Optional finden auch weitere Konstruktionen zur schnellen Aufnahme und Verteilung von Körperflüssigkeit (4) im Saugkern Anwendung. Diese Konstruktionen werden häufig, aber nicht zwingend zwischen der körperzugewandten flüssigkeitsdurchlässigen Abdeckung (1) und der flüssigkeitsabsorbierenden Sauglage (2) eingesetzt.

Die flüssigkeitsdurchlässige Abdeckung (1) besteht in der Regel aus einem nichtgewebten, faserigen Vlies oder einer anderen porösen Konstruktion.
Als Materialien für diese Abdeckung (1) kommen z. B. synthetische Polymere wie etwa Polyvinylchlorid oder -fluorid, Polytetrafluorethylen (PTFE), Polyvinylalkohole und -Derivate, Polyacrylate, Polyamide, Polyester, Polyurethane, Polystyrol, Polysiloxane oder Polyolefine (z.B. Polyethylen (PE) oder Polypropylen (PP)) sowie natürliche Fasermaterialien sowie beliebige Kombinationen aus den vorgenannten Materialien im Sinne von Mischmaterialien oder Verbundmaterialien oder Copolymerisaten in Frage.

Die flüssigkeitsdurchlässige Abdeckung (1) hat hydrophilen Charakter. Sie kann zudem aus einer Kombination von hydrophilen und hydrophoben Bestandteilen bestehen. Bevorzugt ist in der Regel eine hydrophile Ausrüstung der flüssigkeitsdurchlässigen Abdeckung (1), um schnelle Einsickerzeiten von Körperflüssigkeit in die flüssigkeitsabsorbierende Sauglage (2) zu ermöglichen, jedoch werden auch partiell hydrophobierte Abdeckungen (1) verwendet.

Flüssigkeitsabsorbierende Sauglage (2):
Die flüssigkeitsabsorbierende Sauglage (2) enthält die erfindungsgemäßen superabsorbierenden Pulver bzw. Granulate und weitere Komponenten aus beispielsweise faserigen Materialien, schaumförmigen Materialien, filmbildenden Materialien oder porösen Materialien sowie Kombinationen von zwei oder mehreren dieser Materialien. Jedes dieser Materialien kann entweder natürlichen oder synthetischen Ursprungs sein oder durch chemische oder physikalische Modifikation von natürlichen Materialien hergestellt worden sein. Die Materialien können hydrophil oder hydrophob sein, wobei hydrophile Materialien bevorzugt sind. Dies gilt insbesondere für solche Zusammensetzungen, die ausgeschiedene Körperflüssigkeiten effizient aufnehmen und in Richtung zu weiter von der Eintrittsstelle der Körperflüssigkeit entfernte Regionen des absorbierenden Kerns transportieren sollen.

Als hydrophile Fasermaterialien sind geeignet z.B. Cellulosefasern, modifizierte Cellulosefasern (z.B. versteifte Cellulosefasern), Polyesterfasern (z.B. Dacron) hydrophiles Nylon oder aber auch hydrophilisierte hydrophobe Fasern, wie z. B. mit Tensiden hydrophilisierte Polyolefine (PE, PP), Polyester, Polyacrylate, Polyamide, Polystyrol, Polyurethane und andere.

Bevorzugt werden Cellulosefasern und modifizierte Cellulosefasern eingesetzt. Kombinationen von Cellulosefasern und/oder modifizierten Cellulosefasern mit synthetischen Fasern wie z. B. PE/PP Verbundmaterialien, sogenannte Bikomponentenfasern, wie sie z.B. zur Thermobondierung von Airlaidmaterialien verwendet werden oder anderen Materialien sind ebenfalls gebräuchlich.
Die Fasermaterialien können in verschiedenen Anwendungsformen vorliegen, z.B. als lose aus einem Luftstrom oder aus wässriger Phase abgeschiedene oder abgelegte Cellulosefasern, als nichtgewebtes Vlies oder als Tissue. Kombinationen verschiedener Anwendungsformen sind möglich.

Optional können neben den erfindungsgemäßen Superabsorbern weitere pulverförmige Substanzen eingesetzt werden, wie z.B. geruchsbindende Substanzen wie Cyclodextrine, Zeolithe, anorganische oder organische Salze und ähnliche Materialien.

Als poröse Materialien und schaumförmige Materialien können z.B. Polymer-schäume eingesetzt werden wie sie in den Schriften DE 44 18 319 A1 und DE 195 05 709 A1 beschrieben sind.

Zur mechanischen Stabilisierung der flüssigkeitsabsorbierenden Sauglage (2) können thermoplastische Fasern (Z.B. Bikomponentenfasern aus Polyolefinen), Polyolefingranulate, Latexdispersionen oder Heißkleber verwendet werden. Optional werden eine oder mehrere Lagen Tissue zur Stabilisierung verwendet.

Die flüssigkeitsabsorbierende Sauglage (2) kann einlagig sein oder aus mehreren Schichten bestehen. Bevorzugt werden Konstruktionen verwendet, die aus hydrophilen Fasern, bevorzugt Cellulosefasern, optional einer Konstruktion zur schnellen Aufnahme und Verteilung von Körperflüssigkeit (4) wie zum Beispiel chemisch versteifte (modifizierte) Cellulosefasern oder Highloftvliese aus hydrophilen oder hydrophilisierten Fasern sowie superabsorbierenden Polymeren bestehen.

Das erfindungsgemäße superabsorbierende Polymer kann dabei homogen in den Cellulosefasern oder den versteiften Cellulosefasern verteilt sein, es kann lagig zwischen den Cellulosefasern oder den versteiften Cellulosefasern eingebracht sein, oder die Konzentration des superabsorbierenden Polymers kann innerhalb der Cellulosefasern oder versteiften Cellulosefasern einen Gradienten aufweisen. Das Verhältnis der Gesamtmenge an superabsorbierendem Polymer und der Gesamtmenge an Cellulosefasern oder den versteiften Cellulosefasern im absorbierenden Saugkern kann zwischen 0 zu 100 und 70 zu 30 % variieren, wobei in einer Ausführungsform lokal, z. B. bei Gradienteneintrag oder schichtweisem Eintrag Konzentrationen von bis zu 100 % Superabsorber erreicht werden können. Derartige Konstruktionen mit Bereichen hoher Konzentrationen von absorbierendem Polymer, wobei der Anteil von Superabsorber in bestimmten Bereichen zwischen 60 und 100 %, am stärksten bevorzugt zwischen 90 % und 100 % liegt, sind beispielsweise auch in der Patentschrift US 5,669,894 beschrieben.

Optional können auch mehrere verschiedene Superabsorber, die sich zum Beispiel in der Sauggeschwindigkeit, der Permeabilität, der Speicherkapazität, der Absorption gegen Druck, der Kornverteilung oder auch der chemischen Zusammensetzung unterscheiden, gleichzeitig eingesetzt werden. Die verschiedenen Superabsorber können miteinander vermischt in das Saugkissen eingebracht werden oder aber lokal differenziert im Absorbent Core platziert werden. Eine solche differenzierte Platzierung kann in Richtung der Dicke des Saugkissens oder der Länge oder Breite des Saugkissens erfolgen.

In der flüssigkeitsabsorbierenden Sauglage (2) befindet sich eine oder mehrere der oben beschriebenen, superabsorbierende Polymere enthaltenden Lagen aus Cellulosefasern oder versteiften Cellulosefasern. In einer bevorzugten Ausführungsform werden Konstruktionen aus Kombinationen von Lagen mit homogenem Superabsorbereintrag und zusätzlich schichtweiser Einbringung verwendet.

Optional werden diese erwähnten Strukturen auch durch weitere Lagen von reinen Cellulosefasern oder versteiften Cellulosefasern an der körperzugewandten Seite und / oder auch der körperabgewandten Seite ergänzt.

Die oben beschriebenen Konstruktionen können sich auch mehrfach wiederholen, wobei es sich um eine Aufeinanderschichtung zweier oder mehrerer gleicher Lagen oder aber auch um Aufeinanderschichtung zweier oder mehrerer unterschiedlicher Konstruktionen handeln kann. Dabei liegen die Unterschiede in wiederum rein konstruktiver Art oder aber im Typ des verwendeten Materials, wie z.B. die Verwendung von in den Eigenschaften differierenden absorbierenden Polymern oder aber verschiedener Zellstoffarten.

Optional sind das gesamte Saugkissen oder aber auch einzelne Lagen der flüssigkeitsabsorbierenden Sauglage (2) durch Lagen von Tissue von anderen Komponenten getrennt oder stehen in direktem Kontakt mit anderen Lagen oder Komponenten.

Exemplarisch können zum Beispiel die Konstruktion zur schnellen Aufnahme und Verteilung von Körperflüssigkeit (4) und die flüssigkeitsabsorbierende Sauglage (2) durch Tissue voneinander getrennt sein oder aber in direktem Kontakt miteinander stehen. Sofern keine separate Konstruktion zur schnellen Aufnahme und Verteilung von Körperflüssigkeit (4) zwischen der flüssigkeitsabsorbierenden Sauglage (2) und der körperzugewandten flüssigkeitsdurchlässigen Abdeckung (1) existiert, sondern der Effekt der Flüssigkeitsverteilung z.B. durch die Verwendung einer speziellen körperzugewandten flüssigkeitsdurchlässigen Abdeckung (1) erreicht werden soll, kann die flüssigkeitsabsorbierende Sauglage (2) ebenfalls optional von der körperzugewandten flüssigkeitsdurchlässigen Abdeckung (1) durch ein Tissue getrennt sein.

Statt Tissue kann optional auch nichtgewebtes Vlies in die flüssigkeitsabsorbierende Sauglage (2) eingebracht werden. Beide Komponenten führen zu dem erwünschten Nebeneffekt der Stabilisierung und Festigung des Absorptionskerns im feuchten Zustand.

Herstellverfahren der flüssigkeitsabsorbierenden Sauglage:
Faserhaltige, superabsorberhaltige, flüssigkeitsverteilende und -speichernde Schichten lassen sich mit einer Vielzahl von Herstellverfahren generieren. Neben den etablierten konventionellen Prozessen, wie Sie vom Fachmann allgemein unter Drumforming mit Hilfe von Formrädern, -taschen und Produktformen und entsprechend angepassten Dosiereinrichtungen für die Rohstoffe zusammengefasst werden, sind moderne etablierte Verfahren wie der Airlaidprozess (z.B. EP 850 615, Sp. 4 Zeile 39 bis Sp. 5 Zeile 29, US 4.640.810) mit allen Formen der Dosierung, Ablage der Fasern und Verfestigung wie Hydrogenbonding (z.B. DE 197 50 890, Sp. 1 Zeile 45 bis Sp. 3 Zeile 50, Thermobonding, Latexbonding (z.B. EP 850 615, Sp. 8 Zeile 33 bis Sp. 9 Zeile 17 und Hybridbonding, der Wetlaid Prozess (z.B. PCT WO 99/49905, Sp. 4 Zeile 14 bis Sp. 7 Zeile 16), Carding-, Meltblown-, Spunblown- Prozesse sowie ähnliche Prozesse zur Herstellung von superabsorberhaltigen Non-Wovens (im Sinne der der Definition der EDANA, Brüssel) auch in Kombinationen dieser Verfahren mit- und untereinander übliche Methoden zur Herstellung von den o.g. Flüssigkeitsspeichern.

Als weitere Verfahren kommen die Herstellung von Laminaten im weitesteten Sinne sowie von extrudierten und coextrudierten, nass- und trocken- sowie nachträglich verfestigten Strukturen in Frage. Eine Kombinationen dieser Verfahren mit- und untereinander ist ebenfalls möglich.

Konstruktionen zur schnellen Aufnahme und Verteilung von Körperflüssigkeit (4):
Eine Konstruktion zur schnellen Aufnahme und Verteilung von Körperflüssigkeit (4) besteht zum Beispiel aus chemisch versteiften (modifizierte) Cellulosefasern oder Highloftvliesen aus hydrophilen oder hydrophilisierten Fasern oder einer Kombination von beidem.
Chemisch versteifte, modifizierte Cellulosefasern können zum Beispiel erzeugt werden aus Cellulosefasern, die durch Vernetzer wie z.B. C₂ - C₈ Dialdehyde, C₂ - Cg Monoaldehyde mit einer zusätzlichen Säurefunktion oder C₂ - C₉ Polycarbonsäuren in einer chemischen Reaktion umgesetzt werden. Spezielle Beispiele sind: Glutaraldehyd, Glyoxal, Glyoxalsäure oder Zitronensäure. Ebenfalls bekannt sind kationisch modifizierte Stärke oder Polyamid-Epichlorhydrinharze (z. B. KYMENE 557H, Hercules Inc., Wilmington , Delaware). Durch die Vernetzung wird eine verdrehte, gekräuselte Struktur erreicht und stabilisiert, die sich vorteilhaft auf die Geschwindigkeit der Flüssigkeitsaufnahme auswirkt.

Flächengewicht und Dichte von absorbierenden Artikeln:
Die absorbierenden Hygieneprodukte können in ihrem Flächengewicht und Dicke und damit der Dichte stark variieren. Typischerweise liegen die Dichten der Bereiche der Absorptionskerne zwischen 0,08 und 0,25 g/cm³. Die Flächengewichte liegen zwischen 10 und 1000 g/m², wobei bevorzugt Flächengewichte zwischen 100 und 600 g/m² realisiert werden (siehe auch US 5,669,894). Die Dichte variiert in der Regel über die Länge des absorbierenden Kerns. Dies tritt als Folge einer gezielten Dosierung der Cellulosefaser- oder versteiften Cellulosefasermenge oder der Menge des superabsorbierenden Polymers ein, da diese Komponenten in bevorzugten Ausführungsformen stärker in den Frontbereich des absorbierenden Einwegartikels eingebracht werden.

Diese gezielte Erhöhung des absorbierenden Materials in bestimmten Regionen des absorbierenden Kerns kann auf andere Weise auch z.B. durch Herstellung eines entsprechend großen, mittels eines Airlaid- oder Wetlaidverfahrens hergestellten Flächengebildes, bestehend aus hydrophilen Cellulosefasern, optional aus versteiften Cellulosefasern, optional aus synthetischen Fasern (z.B. Polyolefinen) sowie aus superabsorbierenden Polymeren und anschließender Rückfaltung oder Übereinanderlegen erreicht werden.

Die erfindungsgemäßen Polymere werden auch in Absorberartikeln eingesetzt, die für die verschiedensten Verwendungen geeignet sind, so z.B. durch Mischen mit Papier oder Fluff oder synthetischen Fasern oder durch das Verteilen der Superabsorber zwischen Substraten aus Papier, Fluff, oder nicht gewebten Textilien oder durch Verarbeitung in Tägermaterialien zu einer Bahn. Des weiteren finden die erfindungsgemäßen Polymere auch überall dort Verwendung, wo wässrige Flüssigkeiten absorbiert werden müssen, wie z.B. bei Kabelummantelungen, in Lebensmittelverpackungen, im Agrarbereich bei der Pflanzenaufzucht und als Wasserspeicher sowie als Wirkstoffträger mit einer zeitlich verzögerten Freisetzung des Wirkstoffes an die Umgebung.

Die erfindungsgemäßen Produkte mit dieser hervorragenden Eigenschaftskombination aus sehr hohen Absorptions- und Retentionswerten, ausgezeichneter Absorption gegen Druck und biologischer Abbaubarkeit können ohne die Verwendung toxikologisch bedenklicher Substanzen hergestellt werden. Gemäß der Erfindung können die Polymere in großtechnischer Weise nach bekannten Verfahren kontinuierlich oder diskontinuierlich mit konstanter Produktqualität produziert werden.

Weiterhin betrifft die Erfindung eine Konstruktion zur Aufnahme von Körperflüssigkeiten, beinhaltend ein erfindungsgemäßes Polymerisat. Bei den vorstehend genannten Konstruktionen handelt es sich vorzugsweise um Saugkörper. In einer anderen Ausführungsform der Konstruktion handelt es sich um Damenbinden, Windeln oder um Inkontinenzprodukte, hierunter sind Windel besonders bevorzugt.

Die Erfindung wird nachfolgend anhand von Beispielen erläutert.

### Beispiele

Beschreibung der in den Beispielen verwendeten Testmethoden:

### Retention (TB)

Zur Bestimmung der Retentionswerte wurde ein Teebeuteltest durchgeführt. Als Prüflösung wurde eine 0,9 %ige NaCl-Lösung verwendet.

0,20g der Prüfsubstanz (abgesiebt zwischen 150 und 850µm) wurden in einen Teebeutel eingeschweißt und für 30 Minuten in die Prüflösung getaucht. Anschließend wurde in einer Zentrifuge, z.B. einer handelsüblichen Wäscheschleuder, für 3 Minuten bei 1400 Upm abgeschleudert. Die Flüssigkeitsaufnahme wurde nach Abzug des Blindwertes (Gewicht eines leeren Teebeutels nach dem Schleudern) gravimetrisch bestimmt und auf 1 g Prüfsubstanz umgerechnet. Der Retentionswert entspricht der aufgenommenen Flüssigkeitsmenge in Gramm je Gramm Prüfsubstanz.

### Absorption gegen einen Druck von 0,3 bzw. 0,7 psi (AAP)

Das Flüssigkeitsaufnahmevermögen gegen einen äußeren Druck (Absorption Against Pressure, AAP) wurde gemäß der Edana-Methode Nr. 442.1-99 bestimmt.

0,90g der Prüfsubstanz (abgesiebt zwischen 150 und 850µm) wurden in einen Testzylinder mit 60,0 mm Innendurchmesser und einem Siebboden (400mesh) eingewogen (Konzentration: 0,032 g/cm²) und gleichmäßig verteilt. Auf die Prüfsubstanz wird ein zylindrisches Gewicht (21 g/cm² = 0,3 psi oder 50 g/cm² = 0,7psi) mit einem Außendurchmesser von 59,2 mm gelegt. In eine Kunststoffschale werden Filterplatten gelegt, die mit einem Filterpapier abgedeckt werden. Die Kunststoffschale wird mit 0,9 %iger NaCl-Lösung gefüllt, bis der Flüssigkeitsspiegel mit der Oberkante der Filterplatten abschließt. Anschließend werden die vorbereiteten Messeinheiten auf die Filterplatten gestellt. Nach einer Quellzeit von 60 Minuten werden die Messeinheiten entnommen und das Gewicht entfernt. Die aufgenommene Flüssigkeitsmenge wird gravimetrisch ermittelt und auf 1 Gramm Prüfsubstanz umgerechnet.

### Extrahierbare Anteile (EA)

Die extrahierbaren Anteile der biologisch abbaubaren Superabsorberharze wurden mittel GPC-Analytik unter folgenden Prüfbedingungen bestimmt.

Säulenmaterial: HEMA BIO 40, Säulenlänge: 300 mm, Säulendurchmesser: 8 mm, Eluent: 0,9 %ige NaCl-Lsg., Flussrate: 1,0 ml/Minute, Temperatur: Raumtemperatur, Injektionsvolumen: 50 µl, Laufzeit 15 Minuten, Kalibriersubstanz: niedrigviskose Carboxymethylcellulose (Finnfix® 4000G)

Für die Bestimmung der extrahierbaren Anteile wurden 0,50g der Prüfsubstanz mit 100 ml einer 0,9 %igen NaCl-Lösung versetzt und 16 Stunden gerührt. Nach Filtration über einen Glasfiltertiegel (Porenweite 1) wurde das Filtrat im Verhältnis 1:10 mit dem Eluenten verdünnt. Diese Verdünnung wurde injiziert und der Flächenwert des Polymerpeaks bestimmt. Der lösliche Anteil der Prüfsubstanz wurde mit Hilfe einer Kalibrierkurve berechnet, die unter den identischen Bedingungen mit der niedrigviskosen Carboxymethylcellulose erstellt wurde.

### Fluff-Absorber-Combination-Test (FACT)

Auf der Analysenwaage wurden 2,0 g Cellulosefluff eingewogen, aus denen drei Flufflagen geformt wurden. Zwischen die Flufflagen wurden 0,22 bis 2 g (=10 bis 50 Gew.-%), Superabsorberharz gleichmäßig so eingestreut, dass ein "Sandwich" Fluff/SAP/Fluff/SAP/Fluff entsteht. Das Fluff/Superabsorber-Pad wurde in eine Testapparatur mit Siebboden gelegt und mit einem Metallring beschwert um ein Herausquellen des Absorberharzes zu verhindern. Der Prüfling wurde mit einem Gewicht (21 g/cm² bzw. 50 g/cm² entsprechend 0,3 psi bzw. 0,7 psi) belastet. Anschließend ließ man den Prüfling durch Kapillarwirkung in 0,9 %iger NaCl-Lösung quellen und die Absorption mittels elektronischer Datenverarbeitung aufgezeichnet. Der Test galt als beendet, wenn weniger als 1 g Testflüssigkeit innerhalb von 10 Minuten aufgenommen wurde. Von jeder Messung wurde die aufgenommene Flüssigkeitsmenge gegen die Zeit in einer Absorptionskurve dargestellt, aus der folgende Kenngrößen bestimmt wurden:
a) maximal erreichter Endwert in Gramm: Absₘₐₓ
b) Zeit [min], zu dem der Endwert erreicht wurde: tₘₐₓ
c) Zeit [min], zu dem x % des Endwertes erreicht wurden: t_{x%}

### Airlaid Prüfungen

Auf einer Airlaidmaschine wurden Compositematerialien, bestehend aus einer Lage Tissue, einer darauffolgenden Lage aus einer Cellulosefluff/Absoberpulver-Mischung und einer weiteren Lage Tissue, einem pulverförmigen gefertigt.

Aus dem Composite wurden runde Stanzlinge mit 6cm Durchmesser ausgestanzt und für die weiteren Tests verwendet.

### Retention

Zur Bestimmung der Composite-Retentionswerte wurde ein Teebeuteltest durchgeführt. Als Prüflösung wurde eine 0,9 %ige NaCl-Lösung verwendet.

Ein Composite-Stanzling wurde gewogen, in einen Teebeutel eingeschweißt und für 30 Minuten in die Prüflösung getaucht. Anschließend wurde in einer Zentrifuge, z.B. einer handelsüblichen Wäscheschleuder, für 3 Minuten bei 1400 Upm abgeschleudert. Die Flüssigkeitsaufnahme wurde nach Abzug des Blindwertes (Gewicht eines leeren Teebeutels nach dem Schleudern) gravimetrisch bestimmt und auf 1m² Composite umgerechnet. Der Retentionswert entspricht der aufgenommenen Flüssigkeitsmenge in Gramm je Quadratmeter Airlaidcomposite.

### Absorption eines Composites gegen einen Druck von 20 bzw. 50 g/cm² (LAUL20 / LAUL50)

Ein Composite-Stanzling wurde in einen Testzylinder mit 60,0 mm Innendurchmesser und einem Siebboden (400 mesh) eingewogen. Auf die Prüfsubstanz wird ein zylindrisches Gewicht (20 g/cm² oder 50 g/cm²) mit einem Außendurchmesser von 59,2 mm gelegt. In eine Kunststoffschale werden Filterplatten gelegt, die mit einem Filterpapier abgedeckt werden. Die Kunststoffschale wird mit 0,9 %iger NaCl-Lösung gefüllt, bis der Flüssigkeitsspiegel mit der Oberkante der Filterplatten abschließt. Anschließend werden die vorbereiteten Messeinheiten auf die Filterplatten gestellt. Nach einer Quellzeit von 60 Minuten werden die Messeinheiten entnommen und das Gewicht entfernt. Die aufgenommene Flüssigkeitsmenge wird gravimetrisch ermittelt und auf 1 Quadratmeter Airlaidcomposite umgerechnet.

### Mechanische Stabilität

127g eines Mahlmittels (zylindrische Porzellanstücke, U.S. Stoneware ½" O.D. * ½") sowie 10 g eines pulverförmigen Superabsorberharzes mit einer Korngröße von 150 bis 850 µm wurden in einen Kugelmühlentopf eingewogen. Der Kugelmühlentopf wurde verschlossen und für 6 Minuten auf einer Walzenmühle bei 95 Upm rotiert. Der mechanisch belastete Superabsorber wurde dem Topf entnommen und hinsichtlich der Kornverteilung analysiert.

### Oberflächen-Vemetzungungs-Index (Surface-Crosslinking-Index SCI)

127 g eines Mahlmittels (zylindrische Porzellanstücke, U.S. Stoneware ½" O.D. * ½") sowie 10 g eines oberflächenvernetzten Superabsorberharzes mit einer Korngröße von 150 bis 850 µm wurden in einen Kugelmühlentopf eingewogen. Der Kugelmühlentopf wurde verschlossen und für 30 Minuten auf einer Walzenmühle bei 95 Upm rotiert. Der mechanisch belastete Superabsorber wurde dem Topf entnommen und die Partikel mit einer Korngröße <150 µm herausgesiebt. Die abgesiebten Feinkornanteile wurden mit HNO₃ und H₂O₂ mit Hilfe eines Mikrowellengerätes aufgeschlossen und anschließend mit Wasser hydrolysiert. Anschließend wurde der Aluminiumgehalt über den gelbroten Alizarin S-Aluminum-Komplex photometrisch bestimmt. Der SCI berechnet sich aus der, bei der oberflächigen Vernetzung zugegebenen Al³⁺ -Menge bezogen auf das Superabsorberharz (=C_{SAP}) und der, nach mechanischer Belastung ermittelten Al³⁺-Konzentration der Feinpartikel (=C_{F}) nach SCI = (C_{F} - C_{SAP}) *100 wobei C_{F} und C_{SAP} in % Al³⁺ eingesetzt werden.

### Ausführungsbeispiele und Vergleichsbeispiele:

Alle erfindungsgemäßen vorbehandelten Superabsorberharze wurden, wenn nicht anders beschrieben vor der Oberflächenbeschichtung gemahlen und auf eine Korngröße von 150-850 µm abgesiebt. Der Feuchtegehalt aller pulverförmigen Absorberharze betrug weniger als 10 Gew.-%.

### Referenzbeispiel 1

In einem Ansatzkessel wurden 100 g Carboxymethylcellulose (CMC) in eine Mischung aus 244 g 2-Propanol und 156 g E-Wasser suspendiert und unter Rückfluss für 1 Stunde erhitzt. Nach Abkühlen der Suspension auf Raumtemperatur wurde die Carboxymethylcellulose abfiltriert. In einem zweiten Ansatzkessel wurden 900 g Wasser vorgelegt und mit NaOH auf pH 9 eingestellt. Die abfiltrierte Carboxymethylcellulose wurde unter kräftigem Rühren in den zweiten Ansatzkessel eingebracht, wobei sich ein festes Hydrogel bildete. Nach 30 Minuten Quellzeit wurde das gequollene Hydrogel einem Fleischwolf mit Lochscheibe zugeführt und zerkleinert. Das zerkleinerte Hydrogel wurde in einem Umluftschrank bei 80°C für 12 Stunden getrocknet. Das getrocknete Hydrogel wurde grob zerkleinert und mit einer Retschmühle gemahlen. Nach Absiebung der Kornfraktion von 150 bis 850 µm wurde die Retention des unvernetzten Vorproduktes bestimmt. In der folgenden Tabelle sind die so ermittelten Retentionswerte für verschiedene, am Markt erhältliche Carboxymethylcellulosen (CMC) aufgeführt:

| Probe-Nr. | CMC | Viskosität [mPas] | D.S.^{[a]} | Vorprodukt Retention |
|---|---|---|---|---|
| [g/g] | | | | |
| 1.1 | Finnfix® 50.000^{[b]} | 8.200 (1%ig) | 0,78 | 46,5 |
| 1.2 | Cekol® 50.000^{[b]} | 8.400 (1%ig) | 0,72 | 47,0 |
| 1.3 | Cekol® 100.000^{[b]} | 10.000 (1%ig) | 0,76 | 32,2 |
| 1.4 | Tylose® CB30.000^{[c]} | >24.000 (2%ig) | >0,85 | 45,3 |
| 1.5 | Blanose® 7HOF^{[d]} | 2140 (1%ig) | 0,85 | 36,8 |
| 1.6 | Walocel® VP-C-2204^{[e]} | >7500 (1%ig) | 0,65-0,95 | 44,7 |

| | | | | |
|---|---|---|---|---|
| [a]: Substitutionsgrad gemäß Herstellerangaben, [b]: Fa. Noviant [c]: Fa. Clariant, [d]: Fa. Aqualon, [e]: Fa. Wolff-Walsrode | | | | |

### Referenzbeispiel 2

Es wurde eine Vernetzerlösung aus 1,29 g Citronensäure-Monohydrat, 61 g 2-Propanol und 39 g E-Wasser hergestellt. Je 10 g der gemäß Beispiel 1) hergestellten pulverförmigen Vorprodukte wurden mit jeweils 4g dieser Vernetzerlösung beschichtet (entsprechend einer Citronensäurekonzentration von 0,47 % bez. CMC) und für 2 Stunden bei 80°C getrocknet. Die Oberflächenvernetzung wurde durch einen nachfolgenden Temperschritt für den angegebenen Zeitraum auf 120°C abgeschlossen. Die Temperzeit wurde dabei so gewählt, dass ein ausgewogenes Verhältnis von Retention zu Absorption gegen Druck resultiert. Die so hergestellten Superabsorber wiesen folgende Kenndaten auf:

| Nr. | Vorprodukt aus Beispiel | Temperung | TB | AAP_{0,3} | AAP_{0,7} |
|---|---|---|---|---|---|
| | | [min] | [g/g] | [g/g] | [g/g] |
| 2.1 | 1.1 | 30 | 24,0 | 21,6 | 14,4 |
| 2.2 | 1.2 | 50 | 21,0 | 20,5 | 16,1 |
| 2.3 | 1.3 | 30 | 19,4 | 20,9 | 16,8 |
| 2.4 | 1.4 | 30 | 20,4 | 21,8 | 17,2 |

### Beispiel 3

Es wurden verschiedene Vernetzerlösungen durch Zugabe von Aceton zu einer wässrigen Lösung von Aluminiumsulfat-18-Hydrat in E-Wasser hergestellt:
A: 13 g Al₂(SO₄)₃*18 H₂O / 100 g E-Wasser / 36,7 g Aceton
B: 18 g Al₂(SO₄)₃*18 H₂O / 100 g E-Wasser / 36,1 g Aceton

Je 10 g der Vorprodukte aus Beispiel 1 wurden mit je 4 g der so hergestellten Vernetzerlösungen beschichtet, indem das pulverförmige Vorprodukt vorgelegt wurde und unter Rühren die Vernetzerlösung zugetropft wurde. Das beschichtete Produkt wurde bei 80°C für 2 Stunden getrocknet und die getrockneten Produkte hinsichtlich ihrer Retentions- und AAP-Werte untersucht:

| Nr. | Vorprodukt aus Beispiel | Vernetzer-Lösung | %Al³⁺/ CMC | TB [g/g] | AAP_{0,3} [g/g] | AAP_{0,7} [g/g] |
|---|---|---|---|---|---|---|
| 3.1 | 1.1 | B | 0,38 | 24,1 | 18,9 | 13,6 |
| 3.2 | 1.2 | A | 0,28 | 22,6 | 20,5 | 16,1 |
| 3.3 | 1.3 | B | 0,38 | 18,3 | 17,9 | 14,8 |
| 3.4 | 1.6 | B | 0,38 | 22,8 | 18,6 | 14,1 |

### Vergleichsbeispiel 1

20 g Carboxymethylcellulose (Cekol® 50.000, Neutralisationsgrad 98,6%), wurden ohne vorherige Quellung mit 8g einer Lösung von 40g Al₂(SO₄)₃*18 H₂O in 100g E-Wasser und 21,6 g Aceton (0,8 % Al³⁺ bez. CMC) wie in Beispiel 3) beschichtet und für 2 Stunden bei 80°C getrocknet. Anschließend wurden die Absorptionskenndaten bestimmt:

| | | |
|---|---|---|
| Proben-Nr.V 1.1: | TB = 21,1 g/g | AAP_{0,7} = 10,9 g/g |

Im Gegensatz zu der erfindungsgemäß vorbehandelten und nachvernetzten Probe Nr. 3.2, musste im Vergleichsbeispiel 1) eine deutlich höhere Vernetzermenge (0,8 % Al³⁺ bez. CMC statt 0,28 %) eingesetzt werden, obwohl beide Proben auf dem identischen Ausgangsmaterial basieren. Die Absorption gegen Druck (0,7 psi) der erfindungsgemäßen Probe Nr. 3.2 ist mit 16,1 g/g signifikant höher als bei der Vergleichsprobe Nr. V 1.1. Durch die Vorbehandlung wird die Oberfläche der erfindungsgemäßen Absorber derart modifiziert, dass eine effektivere Verteilung und Wirkung des Nachvernetzungsmittels und ein signifikant verbessertes Absorptionsvermögen gegen Druck resultiert.

### Referenzbeispiel 4

Carboxymethylcellulose (Finnfix® 50.000) wurde, wie in Beispiel 1) beschrieben vorgequollen und getrocknet. 50 g des so hergestellten unvernetzten Vorproduktes wurden in einen Plastikbecher eingewogen und mit einem Haushaltsmixer gerührt. Innerhalb von 10 Sekunden wurden 3g einer Lösung, bestehend aus 3,33g Citronensäure Monohydrat und 17,5g Polyethylenglycol (1500 g/mol) in 29,2g E-Wasser, auf das pulverförmige Vorprodukt aufgegeben und für weitere 100 Sekunden nachgerührt. Der beschichtete Absorber wurde für 45 Minuten bei 120°C vernetzt und zeigte folgende Kenndaten:

| | | |
|---|---|---|
| Proben-Nr. 4.1: | TB = 22,2 g/g | AAP_{0,7} = 13,3 g/g |

### Referenzbeispiel 5

Es wurde wie in Beispiel 4) verfahren, nur das für die Beschichtung 3,0 g einer Lösung, zusammengesetzt aus 5,5 g Polyacrylsäure (M_{W} 1500g/mol) und 0,39 g Natriumhydroxid in 10,0 g E-Wasser verwendet wurden:

| | | |
|---|---|---|
| Proben-Nr. 5.1: | TB = 23,2 g/g | AAP_{0,7} = 13,0 g/g |

### Referenzbeispiel 6

Carboxymethylcellulose (Finnfix® 50.000) wurde mit den angegebenen Mengen Guarkernmehl vermischt. Die Pulvermischung wurde wie in Beispiel 1) beschrieben vorgequollen und getrocknet. Jeweils 10 g der getrockneten pulverförmigen Vorprodukte werden, mit 4g einer Lösung von 0,85 g Citronensäure Monohydrat in 99,15 g 2-Propanol beschichtet und für 2 Stunden für 80°C getrocknet. Die Vernetzungsreaktion wurde dann für 30 Minuten bei 120°C vervollständigt.

| Probe Nr. | Gew. % Guarkernmehl | TB [g/g] | AAP_{0,3} [g/g] | AAP_{0,7} [g/g] |
|---|---|---|---|---|
| 6.1 | 5 | 22,4 | 19,1 | 12,7 |
| 6.2 | 10 | 22,5 | 17,5 | 13,1 |
| 6.3 | 20 | 16,2 | 16,5 | 12,8 |

### Referenzbeispiel 7

In einem Ansatzkessel wurden 600 g Carboxymethylcellulose (Cekol® 50.000, Neutralisationsgrad 98,6%) in eine Mischung aus 1460 g 2-Propanol und 940 g E-Wasser suspendiert und für 1 Stunde unter Rückfluss erhitzt. Die Suspension wurde auf Raumtemperatur abgekühlt und filtriert. In einem zweiten Ansatzkessel wurden 5000 g E-Wasser vorgelegt und mit 2,5 g 10%iger Natronlauge auf pH 9 eingestellt. Der Filterkuchen wurde unter starkem Rühren in den zweiten Ansatzkessel eingetragen und das gebildete Hydrogel nach 1 Stunde in einem Fleischwolf mit Lochscheibe zerkleinert. Das zerkleinerte Hydrogel wurde in zwei Hälften geteilt und auf Drahtsieben bei unterschiedlichen Temperaturen getrocknet. Die getrockneten Hydrogele wurden grob zerkleinert, mit einer Retschmühle gemahlen und auf eine Korngröße von 150 bis 850µm abgesiebt. Von den abgesiebten, pulverförmigen Absorber wurde die Retention bestimmt.

In einem weiteren- Versuchsdurchgang wurde der pH-Wert im Ansatzkessel mit 11,5 g 10%iger Natronlauge auf pH 11,3 eingestellt. Die daraus resultierenden Produkte wurden gemäß Beispiel 2 mit der Vernetzungslösung auf Basis von Citronensäure beschichtet und für 50 Minuten bei 120°C nachvernetzt.

| Probe Nr. | pH | Trocknung [°C/h] | TB [g/g] | TB [g/g] | AAP_{0,3} [g/g] | AAP_{0,7} [g/g] |
|---|---|---|---|---|---|---|
| 7.1 | 9 | 80/12 | 45,2 | | | |
| 7.2 | 9 | 150/2,25 | 10,6 | | | |
| 7.3 | 11 | 80/12 | 45,2 | 21,0 | 20,5 | 16,1 |
| 7.4 | 11 | 150/2,25 | 55,6 | 28,2 | 20,5 | 16,0 |

Das Beispiel zeigt den Einfluss von Trocknungstemperatur und pH-Wert auf die interne Vernetzung sowie die Oberflächenverhornung der CMC. Probe 7.2 ist bei hohen Trocknungstemperaturen von 150°C intern vernetzt und zeigt niedrige Retention. Bei Absenkung der Trocknungstemperatur (Probe 7.1) bleibt die interne Vernetzung aus. Durch Anheben des pH-Wertes kann die interne Vernetzung bei hohen Temperaturen verhindert werden wie Probe 7.4 zeigt. Der Vergleich von Probe 7.3 mit 7.4 demonstriert den Einfluss der hohen Temperatur auf die Eigenschaften der erfindungsgemäßen Absorberharze. Es wird angenommen, dass im Verlauf der Trocknung bei hohen Temperaturen eine Verhornung der Oberfläche eintritt, die zu vergleichbaren Absorptionsleistungen gegen Druck bei signifikant höherem Retentionsniveau führt.

Um die interne Vernetzung zu unterbinden, wurde im weiteren, sofern nicht anders beschrieben, der pH-Wert des Quellmediums für die Vorbehandlung derart angepasst, dass bei Trocknungstemperaturen von 150°C eine Vorproduktretention von mindestens 40 g/g erzielt wurde die auch bei Temperung des trockenen Vorproduktes für 60 Minuten bei 120°C nicht unter 40 g/g abfiel.

### Referenzbeispiel 8

In einem Ansatzkessel wird demineralisiertes Wasser vorgelegt und mit unterschiedlichen Mengen 2-Propanol vermischt. Der pH-Wert des Lösungsmittels wird mit 4,7 g 10%iger NaOH pro 1000 ml auf pH 11,7 eingestellt und unter kräftigem Rühren wird Carboxymethylcellulose (Gekol® 100.000, Neutralisationsgrad 98,6%) zugegeben, so dass die Endkonzentration der Carboxymethylcellulose bezogen auf den gesamten Ansatz zwischen 8 und 20 Gew. % liegt. Nach 1 Stunde Quellzeit wurde das gequollene Hydrogel einem Fleischwolf mit Lochscheibe zugeführt und zerkleinert. Das zerkleinerte Hydrogel wurde in einem Umluftschrank bei 150°C für 2,5 Stunden getrocknet. Das getrocknete Hydrogel wurde grob zerkleinert und mit einer Retschmühle gemahlen. Nach Absiebung der Kornfraktion von 150 bis 850µm wurden jeweils 50g des pulverförmigen Vorproduktes in einer Plastikschale vorgelegt, mit einem Mixer gerührt und innerhalb von 10 Sekunden 4,0g einer 50%igen Aluminiumsulfat ×14H₂O-Lösung in E-Wasser, entsprechend 0,36% Al³⁺ bezogen auf CMC aufgesprüht. Das beschichtete Pulver wurde für weitere 110 Sekunden gerührt und anschließend bei 150°C für 10 Minuten getrocknet. Anschließend wurden die Absorptionskenndaten der getrockneten, oberflächenvernetzten Absorber bestimmt.

| Probe Nr. | 2-Propanol im Lösemittel | CMC im Ansatz | TB | AAP_{0,3} | AAP_{0,7} | Schüttdichte |
|---|---|---|---|---|---|---|
| | [Gew. %] | [Gew. %] | [g/g] | [g/g] | [g/g] | [g/dm³] |
| 8.1 | 19,8 | 8 | 28,2 | 20,5 | 16,0 | 450 |
| 8.2 | 13,8 | 12 | 23,2 | 17,5 | 13,5 | 490 |
| 8.3 | 7,1 | 16 | 27,3 | 16,7 | 13,2 | 550 |
| 8.4 | 5 | 20 | 28,5 | 16,4 | 13,4 | 570 |
| 8.5 | 3 | 20 | 28,8 | 16,0 | 13,2 | 590 |
| 8.6 | 1 | 20 | 28,8 | 16,0 | 12,8 | 610 |
| 8.7 | 0 | 20 | 27,7 | 14,4 | 12,5 | 650 |

Beispiel 8 zeigt, dass schon weniger als 5 % Isopropanol im Quellmedium die Schüttdichte der erfindungsgemäßen Absorber reduzieren und Absorption gegen Druck deutlich verbessern. Die Ergebnisse lassen den Schluss zu, dass die rasche Verdampfung des Lösungsmittels bei der Trocknung der Vorprodukte eine zunehmend porösere Kornstruktur zur Folge hat, die sich vor allem positiv auf die Absorption gegen Druck auswirkt.

### Referenzbeispiel 9

4800 g E-Wasser werden in einem Ansatzkessel vorgelegt und mit Natriumhydroxid versetzt (je 1000 ml Wasser enthalten 4,7 g 10%ige Natronlauge) bis der pH-Wert 12 beträgt. Unter Rühren werden 1200 g Carboxymethylcellulose (Cekol ® 100.000, Neutralisationsgrad 98,6%, NaCl-Gehalt 0,74 Gew.-%) zugegeben wobei ein festes Hydrogel gebildet wird. Nach 2 Stunden Quellzeit wurde das Hydrogel in einen Wolf mit Lochscheibe überführt und zerkleinert. Das zerkleinerte Hydrogel wurde 2 Stunden bei 150°C getrocknet, grob zerkleinert und mit einer Retschmühle gemahlen. Die Kornfraktion von 150 bis 850 µm wurde abgesiebt und die Vorproduktkenndaten bestimmt:

| | | | |
|---|---|---|---|
| Proben-Nr. 9.1: | TB = 54,8 g/g | AAP_{0,3} = 8,6 g/g | AAP_{0,7} = 8,3 g/g |

50 g des abgesiebten Vorproduktes Nr. 9.1 wurden vorgelegt, unter Rühren mit 6 g einer 50 %igen Lösung von Al₂(SO₄)₃×14 H₂O in E-Wasser beschichtet (0,54 % Al³⁺ bez. auf CMC) und für 10 Minuten bei 150°C getrocknet:

| | | | |
|---|---|---|---|
| Proben-Nr. 9.2: | TB = 27,7g/g | AAP_{0,3} = 14,4g/g | AAP_{0,7} = 12,5g/g |

50 g des abgesiebten Vorproduktes Nr. 9.1 wurden vorgelegt, unter Rühren mit 8 g einer Lösung von 16,67 g Citronensäure-Monohydrat und 8,33g Natriumhypophosphit in 25 g einer 37,5%igen Lösung von Polyethylenglykol 1500 in E-Wasser (5,1 % Citronensäure, 2,6 % Na-Hypophosphit, 3 % PEG bezogen auf CMC) beschichtet und für 20 Minuten bei 150°C nachvernetzt.

| | | | |
|---|---|---|---|
| Proben-Nr. 9.3: | TB = 23,0 g/g | AAP_{0,3} = 14,3 g/g | AAP_{0,7} = 11,6 g/g |

50 g des abgesiebten Vorproduktes Nr. 9.1 wurden vorgelegt, unter Rühren mit 7 g einer Lösung, zusammengesetzt aus 67 Gew. % einer 50 %igen Al₂(SO₄)₃×14 H₂O-Lösung in E-Wasser und 33 Gew. % einer 40%igen Citronensäure-Monohydratlösung in E-Wasser (0,43 % Al³⁺ und 1,7% Citronensäure bez. auf CMC) beschichtet, und für 20 Minuten bei 140°C nachvernetzt:

| | | | |
|---|---|---|---|
| Proben-Nr. 9.4: | TB = 26,3g/g | AAP_{0,3} = 14,3g/g | AAP_{0,7} = 12,1g/g |

### Referenzbeispiel 10

Jeweils 0,5 g der vorgequollenen, unvernetzten Carboxymethylcellulose Nr. 9.1 sowie der unterschiedlich oberflächenvernetzten Absorber Nr. 9.2 und 9.3 wurden in 100 ml einer 0,9%igen NaCl-Lösung überführt und für 16 Stunden bei Raumtemperatur gerührt. Anschließend wurden die Anteile an Extrahierbarem mittels GPC-Chromatographie bestimmt. Der unbehandelte Rohstoff zeigt bei dieser Analyse eine Löslichkeit von größer 80%.

| Proben-Nr. | 9.1 | 9.2 | 9.3 |
|---|---|---|---|
| Extrahierbare Anteile (%) | 42 | 30 | 21 |

Schon die erfindurigsgemäße Vorbehandlung bewirkt deutlich reduzierte Löslichkeit des Vorproduktes im Vergleich zum verwendeten Rohstoff. Durch die nachfolgende Oberflächenvernetzung wird die Löslichkeit nochmals gesenkt.

### Referenzbeispiel 11

A: Eine pulverförmige Carboxymethylcellulose (Cekol® 100.000, Neutralisationsgrad 98,6%) wurde mit Wasser bei unterschiedlichen pH-Werten in einem Doppelschneckenextruder coextrudiert. Der Gesamtdurchsatz betrug 56 kg/Stunde und der Anteil Carboxymethylcellulose im Hydrogel betrug 20-25 Gew. %. Die pH-Regulierung des wässrigen Lösungsmittels erfolgte durch Zugabe von Natriumhydroxid. Die Extruderwellen wurden mit zusätzlichen Knetelementen ausgerüstet, um die Homogenität des Hydrogeles zu verbessern. Das gebildete Hydrogel wurde durch eine Lochscheibe gepresst, die erhaltenen Gelstränge bei 150°C getrocknet und anschließend gemahlen und auf 150-850 µm abgesiebt. Das gesiebte, pulverförmige Vorprodukt wurde hinsichtlich seiner Retention analysiert:

| Proben-Nr. | pH-Wert des Quellmediums | TB [g/g] |
|---|---|---|
| 11.1 | 7 | 42,9 |
| 11.2 | 8 | 41,8 |
| 11.3 | 9 | 41,0 |
| 11.4 | 10 | 41.1 |

B: Es wurde wie unter A verfahren, jedoch ohne die Verwendung von Knetelementen bei einem Gesamtdurchsatz von 99 kg Hydrogel pro Stunde. Die Homogenität des Gels war sichtbar geringer als bei A. Vereinzelt enthielten die Gelstränge trockene Partikel.

| Proben-Nr. | pH-Wert des Quellmediums | TB [g/g] |
|---|---|---|
| 11.5 | 7 | 43,1 |
| 11.6 | 8 | 45,2 |
| 11.7 | 9 | 42,7 |
| 11.8 | 10 | 41,6 |

C: Es wurde wie unter B verfahren, jedoch wurde bei einem Gesamtdurchsatz von 97-102 kg Hydrogel pro Stunde der Carboxymethylcelluloseanteil im Hydrogel zwischen 20 und 45 Gew. % variiert. Der pH-Wert des Quellmediums wurde in allen Fällen mit Natriumhydroxid auf pH 7,5 eingestellt:

| Proben-Nr. | Anteil CMC im Hydrosel [Gew.-%] | TB [g/g] |
|---|---|---|
| 11.9 | 23 | 44,5 |
| 11.10 | 29 | 41,7 |
| 11.11 | 35 | 43,1 |
| 11.12 | 40 | 45,2 |
| 11.13 | 45 | 42.4 |

Jeweils 50 g der pulverförmigen Vorprodukte aus A, B und C wurden in einem Mischreaktor vorgelegt. Unter Rühren wurde die Oberfläche der Vorprodukte mit jeweils 5 g einer 50%igen Aluminiumsulftat-14-Hydrat-Lösung beschichtet. Die beschichteten Produkte wurden jeweils bei 120°C für 20 Minuten getrocknet und bezüglich ihrer Retentionswerte und ihrer Absorption gegen Druck analysiert:

| Proben-Nr. | Vorprodukt-Nr. | TB [g/g] | AAP_{0,3} [g/g] | AAP_{0,7} [g/g] |
|---|---|---|---|---|
| 11.14 | 11.1 | 25,4 | 16,0 | 12,6 |
| 11.15 | 11.4 | 30,2 | 15,1 | 11,9 |
| 11.16 | 11.5 | 27,0 | 15,9 | 12,7 |
| 11.17 | 11.6 | 31,4 | 15,9 | 12,6 |
| 11.18 | 11.7 | 29,4 | 15,3 | 11,6 |
| 11.19 | 11.11 | 34.4 | 15,9 | 12,9 |

Die Ergebnisse belegen, dass die erfindungsgemäßen Absorber nach kontinuierlichen Verfahren in großem Maße zugänglich sind, wobei der Extrusionsprozess keinerlei Einschränkungen erkennen lässt. Selbst weniger homogene Gelstränge, die z.B. bei Extrusion mit hohem Durchsatz und geringem Wasseranteil ohne die Verwendung von Knetelementen resultieren, führen zu keiner Beeinträchtigung in den Absorptionseigenschaften der erfindungsgemäßen Absorberharze. Weiterhin verdeutlicht Beispiel 11 den gemeinsamen Einfluss des pH-Wertes und der Mischtechnologie auf die interne Vernetzung. Wird, wie hier, ein Mischprozess verwendet, bei dem das Quellmedium dem trockenen Rohstoff kontinuierlich zugeführt wird und zu keinem Zeitpunkt im Überschuss vorliegt, werden schon bei pH = 7 Vorprodukte erhalten, die bei der Trocknung nicht intern vernetzen. Es wird angenommen, dass ein Zusammenhang zwischen dem Solubilisierungsgrad des Rohstoffes und der Reaktivität in Bezug auf die interne Vernetzung besteht. In den vorherigen Beispielen 1 bis 10 wurde das Quellmedium vorgelegt, d. h. die Polysaccharide -lagen zu Beginn der Quellung in größerer Verdünnung vor und konnten eine reaktivere Vorzugskonformation einnehmen. Durch Anheben des pH-Wertes wurden die freien Säurefunktionalitäten neutralisiert und damit der internen Vernetzung entgegengewirkt.

### Vergleichsbeispiel 2

Hier wird in Anlehnung an die US 5,470,964 bzw. US 5,550,189 gearbeitet. In einem Reaktionsgefäß wurden jeweils 200g einer wässrigen Aluminiumsulfatlösung vorgelegt wobei die Konzentration des Aluminiumsulfates × 14 H₂O im Bereich von 0,25 bis 0,75 Gew. % bezogen auf die Lösung variiert wurde. In die Lösungen wurde unter kräftigem Rühren jeweils 50 g Carboxymethylcellulose (Cekol® 100.000, Neutralisationsgrad 98,6%) eingetragen. Die Lösung ging sehr schnell in den Gelzustand über, so das gegen Ende der Zugabe eine homogene Mischung nicht mehr möglich war. Das gequollene Hydrogel wurde für 5 Stunden bei 80°C getrocknet, grob zerkleinert und nach Mahlen mit einer Retschmühle auf 150 bis 850µm abgesiebt. Für die gesiebten Kornfraktionen wurden folgende Kenndaten bestimmt:

| Proben-Nr. | [mg] Al₂(SO₄)₃×14 H₂O / bezogen [g] CMC | [Al³⁺]* / CMC | TB [g/g] | AAP_{0,3} [g/g] | AAP_{0,7} [g/g] |
|---|---|---|---|---|---|
| V2.1 | 10 | 0,09 | 32,8 | 12,3 | 9,9 |
| V2.2 | 15 | 0,14 | 27,9 | 13,5 | 10,7 |
| V2.3 | 20 | 0,18 | 23,7 | 14,1 | 11,7 |
| V2.4 | 40 | 0,36 | 16,3 | 14,4 | 12,4 |
| V2.5 | 70 | 0,63 | 15,3 | 14,0 | 12,0 |
| V2.6# | 15 | 0.14 | 12.3 | 12.5 | 10.2 |

| | | | | | |
|---|---|---|---|---|---|
| V2.6#: Trocknung für 30 Minuten bei 150°C [ ]*: Angabe in Gew. % | | | | | |

Das Vergleichsbeispiel 2 zeigt, dass die Oberflächenvernetzung mit großen Mengen Lösungsmittel, nicht zu dem Eigenschaftsprofil der erfindungsgemäßen Absorberharze führt. Absorptionen gegen einen Druck (0,3 psi) von ≥ 14 g/g sind nur bei Retentionen von weniger als 25 g/g zu erzielen. Retentionswerte ≥25 g/g sind nur bei geringem AAP_{0,7} von weniger als 11 g/g zu realisieren. Höhere Trocknungstemperaturen führen bei Probe Nr. V2.6 zu einer starken Vernetzung und deutlich verschlechterten Absorptionseigenschaften. Der erfindungsgemäße Absorber Nr. 9.2, basierend auf dem gleichen Rohstoff, zeigt dagegen bei einer Retention von 27,7 g/g auch hohe Absorption gegen Druck (14,4 g/g [0,3 psi] bzw. 12,5 g/g [0,7 psi].

### Vergleichsbeispiel 3

Hier wird in Anlehnung an die EP 538 904 B1 bzw. die US 5,247,964 die unter sauren pH-Bedingungen sich einstellende interne Vernetzung der CMC nachgestellt. Carboxymethylcellulose (Cekol® 100000, Neutralisationsgrad 98,6%) wird wie in Beispiel 11) beschrieben coextrudiert. Der pH-Wert des Quellmediums wurde dabei mit Schwefelsäure auf pH 6 eingestellt. Der Feststoffanteil im Hydrogel betrug 23-25 Gew. % bei unterschiedlichem Gesamtdurchsatz. Das gequollene Hydrogel wurde für 60 Minuten bei 150°C getrocknet, wobei die Polymerpartikel wegen des sauren pH-Wertes des Quellmediums intern vernetzten. Die getrockneten Polymerpartikel wurden gemahlen, auf 150-850 µm abgesiebt und hinsichtlich ihrer Absorptionskenndaten analysiert:

| Proben-Nr. | Hydrogel-Gesamtdurchsatz [kg/h] | TB [g/g] | AAP_{0,3} [g/g] | AAP_{0,7} [g/g] |
|---|---|---|---|---|
| V3.1 | 56 | 25,1 | 11,6 | 8,2 |
| V3.2 | 99 | 28,2 | 14,3 | 8.9 |

Das Vergleichbeispiel zeigt, dass die bei sauren pH-Werten extrudierten Vorprodukte nach der Trocknung aufgrund der internen Vernetzung nur Retentionswerte von weniger als 30 g/g aufweisen. Im Gegensatz zu den erfindungsgemäßen Ab-sorberharzen zeigen die intern vernetzten Produkte kein verbessertes Absorptionsvermögen gegen Druck (AAP_{0,7}).

### Beispiel 12

1200g Carboxymethylcellulose (Finnfix® 50.000) wurden wie in Beispiel 9) beschrieben vorgequollen, getrocknet, gemahlen und auf 150-850µm gesiebt. Jeweils 50 g des gesiebten Vorproduktes wurden unter Rühren mit einer 50 %igen Lösung von Al₂(SO₄)₃×14 H₂O in E-Wasser beschichtet und für 20 Minuten bei 120°C getrocknet. Die oberflächig beschichteten Absorber wurden hinsichtlich ihres Surface Crosslinking Index analysiert:

| Proben-Nr. | g 50%ige Al₂(SO₄)₃×14H₂O-Lsg./ 50g Vorprodukt | C_{SAP} [Al³⁺]* | C_{F} [Al³⁺]* | SCI |
|---|---|---|---|---|
| 12.1 | 4,00 | 0,36 | 0,78 | 42 |
| 12.2 | 5,00 | 0,45 | 0,90 | 45 |
| 12.3 | 7,00 | 0,64 | 1.20 | 56 |

| | | | | |
|---|---|---|---|---|
| [ ]*:Angaben in Gew.-% | | | | |

### Vergleichsbeispiel 4

Die in Vergleichsbeispiel 2 hergestellten Absorber wurden hinsichtlich ihres Surface Crosslinking Index analysiert:

| Proben-Nr. | C_{SAP} [Al³⁺] | C_{F} [Al³⁺] | SCI |
|---|---|---|---|
| V4.1 | 0,14 | 0,26 | 12 |
| V4.2 | 0,18 | 0,36 | 18 |
| V4.3 | 0,36 | 0,71 | 35 |
| V4.4 | 0,64 | 0,99 | 35 |

| | | | |
|---|---|---|---|
| [ ]*:Angaben in Gew.% | | | |

### Referenzbeispiel 13

Die Absorption gegen Druck des pulverförmigen Absorberharzes Probe Nr. 11.17 (Beispiel 11) wurde mit dem Fluff-Absorber-Combination-Test im Vergleich zu einem synthetischem Superabsorber (Produkt Z1030, synthetisches, vor- und nachvernetztes Polyacrylsäurepolymer mit Neutralisationsgrad = 70 %, Kenndaten: AAP_{0,3}-31,6 g/g und AAP_{0,7}=24,4 g/g, Fa. Stockhausen GmbH & Co. KG) untersucht. Aus der Absorptionskurve wurden in Abhängigkeit von der Konzentration an Superabsorber (SAP) folgende Kenndaten bestimmt:

| Probe | Kenngröße | FACT bei 0,3 psi | | | FACT bei 0,7 psi | | |
|---|---|---|---|---|---|---|---|
| | | SAP-Konz. im Pad | | | SAP-Konz. im Pad | | |
| | | 10% | 31% | 50% | 10% | 31% | 50% |
| 11.17 | Absₘₐₓ [g/g] | 20,0 | 29,7 | 42,3 | 15,0 | 23,0 | 29,0 |
| | tₘₐₓ [min] | 16 | 27 | 84 | 16 | 25 | 47 |
| | t_{50%} [min] | 1 | 3 | 9 | 1 | 2 | 7 |
| Z1030 | Absₘₐₓ [g/g] | 21,8 | 39,7 | 68,5 | 17,4 | 32,8 | 53,5 |
| | tₘₐₓ [min] | 20 | 29 | 48 | 19 | 36 | 69 |
| | t_{50%} [min] | 2 | 4 | 9 | 2 | 6 | 12 |

Beispiel 13 zeigt, dass sich das Absorptionsvermögen der erfindungsgemäßen Absorberharze durch Kombination mit einem Matrixmaterial relativ zu einem synthetischen Polyacrylatabsorber signifikant verbessert. Während Probe Nr. 11.17 weniger als 50% des Absorptionsvermögens gegen Druck (0,3 oder 0,7psi) eines Z1030 besitzt, steigert sich dieser Prozentwert bei 50% SAP im Fluff / SAP-Gemisch auf ≥ 55% bis hin zu ≥86% bei 10% SAP im Fluff / SAP -Gemisch.

### Referenzbeispiel 14

Das oberflächig vernetzte pulverförmige Absorberharz Probe Nr. 11.15 aus Beispiel 11 wurde mit unterschiedlichen Cellulose-Fluffinengen zu einem Airlaid-Composite-Artikel verarbeitet. Zu Vergleichszwecken wurde ein synthetischer Polyacrylat-Superabsorber (Z 1030, Stockhausen) unter identischen Bedingungen zu einem Composite-Artikel verarbeitet. Die Composites wurden hinsichtlich ihres Retentionsverhaltens und der Flüssigkeitsabsorption gegen einen Druck von 20- bzw. 50g/cm² charakterisiert:

| Airlaid Composites | | | | | | | |
|---|---|---|---|---|---|---|---|
| Fluffanteil im Composite | SAP (Typ) | Retention | | LAUL20 (0,3psi) | | LAUL50 (0,7psi) | |
| | | Absolut [g/m²] | Relativ* | Absolut [g/m²] | Relativ* | Absolut [g/m²] | Relativ* |
| 50% | 11.15 | 6821 | 86,6 | 7784 | 65,0 | 6337 | 67,0 |
| | Z 1030 | 7878 | - | 11963 | - | 9452 | - |
| 70% | 11.15 | 3566 | 82,1 | 5284 | 68,9 | 4314 | 72,2 |
| | Z 1030 | 4345 | - | 7673 | - | 5974 | - |
| 90% | 11.15 | 1219 | 86,7 | 3210 | 81,7 | 2568 | 80,2 |
| | Z 1030 | 1406 | - | 3928 | - | 3199 | - |

| Kenndaten der reinen, pulverförmigen Superabsorberharze | | | | | | | |
|---|---|---|---|---|---|---|---|
| ohne Fluff | SAP (Typ) | Retention | | AAP_{0,3} | | AAP_{0,7} | |
| | | Absolut [g/g] | Relativ* | Absolut [g/g] | Relativ* | Absolut [g/g] | Relativ* |
| - | 11.15 | 30,2 | 97,4 | 15,1 | 47,8 | 11,9 | 48,7 |
| | Z 1030 | 31,0 | - | 31,6 | - | 24,4 | - |

| | | | | | | | |
|---|---|---|---|---|---|---|---|
| *: Produkt Z1030 = 100 | | | | | | | |

Beispiel 14 zeigt die Performanceverbesserung der erfindungsgemäßen Absorber relativ zu synthetischen Superabsorbern in Airlaidcomposites. Durch die homogenere Mischung des Matrixmaterials mit dem Absorber wirkt sich diese relative Leistungssteigerung gerade bei hohen Absorbergehalten in der Matrix noch deutlicher aus als in Beispiel 13.

### Beispiel 15

Zur Charakterisierung der Alterungsstabilität wurden die biologisch abbaubaren Superabsorberharze für einen längeren Zeitraum bei Raumtemperatur und einer durchschnittlichen Luftfeuchtigkeit von mehr als 50 % gelagert und anschließend die Retention und die Absorption gegen Druck gemessen.

| Kenndaten nach der Synthese Kenndaten | | | | nach | | Lagerung | |
|---|---|---|---|---|---|---|---|
| Proben Nr. | TB [g/g] | AAP_{0,3} [g/g] | AAP_{0,7} [g/g] | Alter in Tagen | TB [g/g] | AAP_{0,3} [g/g] | AAP_{0,7} [g/g] |
| 2.3 | 19,4 | 20,9 | 16,8 | 513 | 19,2 | 19,4 | 15,4 |
| 2.4 | 20,4 | 21,8 | 17,2 | 384 | 20,5 | 22,0 | 15,8 |
| 8.1 | 28,2 | 20,5 | 16,0 | 222 | 32,6 | 19,0 | 14,1 |
| 9.2 | 27,7 | 14,4 | 12.5 | 225 | 25,5 | 14.1 | 11,4 |

### Vergleichsbeispiel 5

In Anlehnung an die Lehre der EP 538 904 bzw. US 5,247,072 wurden in einem Ansatzkessel 1980 g E-Wasser vorgelegt und mit NaOH auf einen pH-Wert von 9 eingestellt. Unter Rühren wurden 20 g Carboxymethylcellulose (Blanose® 7HOF, Aqualon) zugegeben. Die 2 %ige Lösung wurde für 20 Stunden bei 80°C getrocknet. Das getrocknete Produkt wurde gemahlen, auf eine Korngröße von 150-850 µm abgesiebt und für weitere 120 Minuten bei 150°C getempert. Von dem Produkt wurden die Absorptionskenndaten direkt nach der Synthese sowie nach Lagerung bei Raumtemperatur und einer Luftfeuchtigkeit von mehr als 50 % bestimmt:

**Proben Nr. V5.1**

| | | |
|---|---|---|
| Nach der Synthese: | TB = 23,6 g/g | AAP_{0,3}= 20,4 g/g |
| Nach 10 Tagen: | TB = 23,9 g/g | AAP_{0,3} = 15,1 g/g |
| Nach 100 Tagen | TB = 26,3 g/g | AAP_{0,3} = 7,4 g/g |
| Nach 200 Tagen: | TB = 29,2 g/g | AAP_{0,3} = 7,2 g/g |

Vergleichsbeispiel 5 zeigt, dass ausgehend von verdünnten CMC-Lösungen durch Trocknung und interne Vernetzung hohe AAP_{0,3}-Werte direkt nach der Synthese zugänglich sind. Die Produkte sind jedoch im Gegensatz zu den erfindungsgemäßen Superabsorbern nicht alterungsstabil und daher schon nach kurzer Zeit nicht mehr als Superabsorber in kommerziellen Produkten verwendbar.

### Beispiel 16

Als Kenngröße für die mechanische Stabilität wurde die Kornverteilung der pulverförmigen Superabsorber vor und nach der Belastung in der Kugelmühle bestimmt. Die Angaben in der folgenden Tabelle beziehen sich auf den Gew. %-Anteil der einzelnen Kornfraktionen:

| Proben-Nr. | Kornfraktion | | | Kornfraktion | | | |
|---|---|---|---|---|---|---|---|
| | ohne | mech. | Belastung | nach | mech. | Belastung | |
| | 150-300µm | 300-600µm | 600-850µm | 150-300µm | 300-600µm | 600 850µm | <150µm |
| 8.1 | 12,853,6 | 33,6 | 16,0 | 55,6 | 27,4 | 1,0 | |
| 11.15 | 19,653,7 | 26,7 | 21,6 | 51,9 | 25,7 | 0,8 | |
| 11.17 | 23,352,2 | 24,5 | 23,5 | 52,6 | 22,5 | 1,3 | |
| 11.19 | 11.061.6 | 27.4 | 24.6 | 50,7 | 23,4 | 1,3 | |

Das Beispiel demonstriert, dass die erfindungsgemäßen Superabsorber mechanisch sehr stabil sind und auch nach einer mechanischen Belastung, wie sie z.B. bei der Produktförderung auftritt, eine ähnliche Kornverteilung und nur sehr geringe Feinkornanteile <150 µm aufweist. Dadurch werden gleichbleibende Produkteigenschaften auch nach Förder- und Dosierprozessen gewährleistet.

### Vergleichsbeispiel 6

Hier wird aus der US 4,043,952 das Beispiel 20 unter Verwendung der Carboxymethylcellulose Blanose H nachgestellt. Die CMC wurde dabei in methanolischer Suspension mit 0,64 mÄqu. Al-Kation pro Gramm CMC umgesetzt. Das Reaktionsprodukt wies die folgenden Kenndaten auf:
Retention: 28,9 g/g, AAP(0.3 psi): 9,2 g/g, AAP(0.7 psi): 7,4 g/g

Das Vergleichsbeispiel macht deutlich, dass die nach der US 4,043,952 in inerten Lösemitteln hergestellten, an der Oberfläche mit polyvalenten Kationen behandelten CMC-Produkte sehr niedrige Absorptionseigenschaften unter Druck aufweisen.

### Vergleichsbeispiel 7

Hier wird das Beispiel 1 der US 5,811,531 nachgestellt, wobei Xanthan Gum mit einer geringen Menge wässrig-methanolischer Ehylenglykoldiglycidylether-Lösung vermischt und bei 140°C erhitz wird. Es ergibt sich ein Produkt mit folgenden Eigenschaften:
Retention: 29,3 g/g, AAP(0.3 psi): 7,9 g/g, AAP(0.7 psi): 5,7 g/g

Auch hier zeigt es sich, das durch das in der US 5,811,531 beschriebene Verfahren Produkte mit schlechten Absorptionswerten unter Druck entstehen.

Die Beispiele zeigen, dass die erfindungsgemäßen Polymere bei einem sehr hohen Retentionsvermögen ein signifikant verbessertes Aufnahmevermögens für Wasser und wässrige Flüssigkeiten entgegen einem äußeren Druck aufweisen. Sie weisen neben einer hohen Langzeitlagerstabilität auch eine gute biologische Abbaubarkeit unter Kompostbedingungen auf. Gezeigt wurde auch, dass sich nur durch das erfindungsgemäße Verfahren, bestehend aus der Herstellung eines Hydrogeles gefolgt von der Trocknung unter Bedingungen, die zu einer Verhornung, aber nicht zu einer internen Vernetzung führen und die anschließende Oberflächenvernetzung in geringer Schichtdicke die einzigartige Eigenschaftskombination aus hohem Retentionsvermögen, hohem Absorptionsvermögen gegen einen äußeren Druck, Lagerstabilität und biologischer Abbaubarkeit erreichen lässt. Weiterhin zeigen insbesondere die Beispiele 19) und 20), dass die erfindungsgemäßen Polymere in einer absorbierenden Konstruktion für die Aufnahme von Körperflüssigkeiten durch die Kombination mit einem Matrixmaterial wie z.B. Cellulose-Fluff eine signifikant höhere Aufnahme von Flüssigkeiten auch bei hohen Absorberharzkonzentrationen in der Konstruktion, vor allem gegen einen äußeren Druck, relativ zu einem synthetischen Absorberharz entwickeln. Die Oberflächenvernetzung eines nicht vorgequollenen Produktes führt nicht zu einer vergleichbaren Verbesserung des Absorptionsvermögens gegen Druck (Vergleichsbeispiel 1, 6 und 7). Auch die interne Vernetzung ausgehend von einem Hydrogel oder einer verdünnten Lösung fuhrt nicht zu dem gewünschten Eigenschaftsprofil (Vergleichsbeispiel 3 und 5). Die Oberflächenvernetzung mit größerer Schichtdicke führt nicht annähernd zu Superabsorbern, die mit den beschriebenen Produkten vergleichbar sind. (Vergleichsbeispiel 2). Vielmehr zeigen die Produkte nur bei deutlich geringerem Retentionsvermögen ein gewisses Absorptionsvermögen gegen Druck. Darüber hinaus treten bei der Oberflächenvernetzung der Polymere mit großer Schichtdicke erhebliche Probleme bezüglich der Durchführbarkeit der Verfahren auf (völliges Verklumpen des Materials und große Inhomogenitäten innerhalb der Mischung).

## Patentansprüche

1. Pulverförmiges, an der Oberfläche nachvernetztes Wasser, wässrige oder seröse Flüssigkeiten sowie Blut absorbierendes Polymerisat, erhältlich durch eine Oberflächenvernetzung mindestens eines teilneutralisierten, Carboxylgruppen enthaltenden Polysaccharids, **dadurch gekennzeichnet, dass** das Polycarboxypolysaccharid vor der Oberflächenvernetzung in unvernetzter Form wässrig vorgequollenen und wieder getrocknet wird, dass zur Oberflächennachvernetzung ionische Vernetzer verwendet werden, dass die ionischen Oberflächenvernetzer Salze mindestens zweiwertiger Kationen sind, dass das mehrwertige Kation aus Al³⁺ gebildet ist, wobei das Polymerisatpulver einen Oberflächen-Vernetzungs-Index (SCI) von größer 40 aufweist, wobei sich der SCI berechnet aus der bei der oberflächigen Vernetzung zugegebenen Al³⁺ -Menge bezogen auf das Superabsorberharz (=C_{SAP}) und der nach mechanischer Belastung ermittelten Al³⁺-Konzentration der Feinpartikel (=C_{F}) nach SCI = (C_{F} - C_{SAP}) *100 wobei C_{F} und C_{SAP} in % Al³⁺ eingesetzt werden.

2. Polymerisatpulver nach Anspruch 1, **dadurch gekennzeichnet, dass** der Oberflächenvernetzer in einer Menge von 0,01 - 25 Gew.-%, bezogen auf das Polycarboxypolysaccharid vorhanden ist.

3. Polymerisatpulver nach Anspruch 1, **dadurch gekennzeichnet, dass** der Oberflächenvemetzer in einer Menge von 0,2 - 1,0 Gew%, bezogen auf das Polycarboxypolysaccharid eingesetzt werden.

4. Polymerisatpulver nach den Ansprüchen 1-3, **dadurch gekennzeichnet, dass** es bei einer Retention von größer oder gleich 20 g/g eine Absorption gegen Druck (AAP_{0,7}) bestimmt nach der Edena-Methode Nr. 442.1-99 gemäß der Beschreibung mindestens 11 g/g aufweist.

5. Polymerisatpulver nach den Ansprüchen 1-3, **dadurch gekennzeichnet, dass** es bei einer Retention von größer oder gleich 25 g/g eine Absorption gegen Druck (AAP_{0,7}) von mindestens 11 g/g aufweist.

6. Polymerisatpulver nach den Ansprüchen 1-3, **dadurch gekennzeichnet, dass** es nach einer Alterung von 200 Tagen unter Normalbedingungen mindestens 80% der Absorption gegen Druck (AAP_{0,7}) verglichen zum Ausgangswert zeigt.

7. Polymerisatpulver nach den Ansprüchen 1-3, **dadurch gekennzeichnet, dass** es nach mechanischer Belastung durch 6minütiges Rollmahlen weniger als 5 Gew. % Feinkornanteil mit einer Korngröße unterhalb 150 µm bildet.

8. Verfahren zur Herstellung von absorbierenden Polymerisaten gemäß einem der Ansprüche 1 bis 3, durch Vernetzen der Oberfläche eines Polycarboxypolysaccharids mit einem Oberflächenvernetzer, **dadurch gekennzeichnet, dass** aus einem unvernetzten Polycarboxypolysaccharid mit Wasser ein Hydrogel gebildet wird, das Hydrogel mechanisch zerkleinert und getrocknet wird, eine Zerkleinerung und Klassierung des getrockneten Hydrogels unter Ausbildung eines Polymerpulvers erfolgt und dass die Partikel des Polymerpulvers mit einer Lösung eines Vernetzers beschichtet und anschließend einer Oberflächennachvernetzung unterworfen werden, dass 0,01 - 25 Gew. %, ionischer Oberflächennachvernetzer, bezogen auf das Polymerpulver, in Form einer 0,01 - 80 Gew. %igen, wässrigen Lösung zugesetzt werden und dass die Oberflächennachvernetzung bei Temperaturen von 40°C bis 250°C über einen Zeitraum von 5 Minuten bis 6 Stunden durchgeführt wird.

9. Verfahren nach Anspruch 8, **dadurch gekennzeichnet, dass** die Mischung aus Polycarboxypolysaccharid und Wasser in einem kontinuierlichen Mischer durchgeführt wird.

10. Verfahren nach Anspruch 8, **dadurch gekennzeichnet, dass** die wässrige Lösung des kovalenten Oberflächennachvernetzers zusätzlich einen Vernetzungskatalysator enthält.

11. Konstruktion zur Aufnahme von Körperflüssigkeiten, beinhaltend ein Polymerisat gemäss einem der Ansprüche 1 - 3 und 5 - 8.

## Claims

1. Pulverulent surface-postcrosslinked addition polymer capable of absorbing water, aqueous or serous fluids and also blood and obtainable by surface crosslinking at least one partly neutralized carboxyl-containing polysaccharide, **characterized in that** the polycarboxypolysaccharide is aqueously preswollen in uncrosslinked form and redried before the surface crosslinking, **in that** the surface postcrosslinking utilizes ionic crosslinkers, **in that** the ionic surface crosslinkers are salts of at least divalent cations, **in that** the polyvalent cation is formed from Al³⁺, wherein the addition polymer powder has a surface crosslinking index (SCI) of greater than 40, where the SCI is calculated from the amount of Al³⁺ added in the course of the surficial crosslinking, based on the superabsorbent resin (= C_{SAP}) , and the Al³⁺ concentration of the fine particles found after mechanical exposure (= C_{F}) in accordance with the equation SCI = (C_{F}-C_{SAP}) *100, where C_{F} and C_{SAP} are inserted in % Al³⁺.

2. Addition polymer powder according to Claim 1, **characterized in that** the surface crosslinker is present in an amount of 0.01-25% by weight, based on the polycarboxypolysaccharide.

3. Addition polymer powder according to Claim 1, **characterized in that** the surface crosslinker is used in an amount of 0.2-1.0% by weight, based on the polycarboxypolysaccharide.

4. Addition polymer powder according to any of Claims 1-3, **characterized in that** it has a retention of not less than 20 g/g coupled with an absorbency against pressure (AAP_{0.7}) value determined according to Edana method No. 442.1-99 according to the description of at least 11 g/g.

5. Addition polymer powder according to any of Claims 1-3, **characterized in that** it has a retention of not less than 25 g/g coupled with an absorbency against pressure (AAP_{0.7}) value of at least 11 g/g.

6. Addition polymer powder according to any of Claims 1-3, **characterized in that** its absorbency against pressure (AAP_{0.7}) value is not less than 80% of the initial value after ageing for 200 days under standard conditions.

7. Addition polymer powder according to any of Claims 1-3, **characterized in that** it will have formed less than 5% by weight of fines having a particle size of below 150 µm after mechanical exposure due to roller milling for 6 minutes.

8. Process for preparing absorbent addition polymers according to any of Claims 1 to 3 by crosslinking the surface of a polycarboxypolysaccharide with a surface crosslinker, **characterized in that** a hydrogel is formed from an uncrosslinked polycarboxypolysaccharide with water, mechanically comminuted and dried, the dried hydrogel is comminuted and classified to form a polymer powder and **in that** the particles of the polymer powder are coated with a solution of a crosslinker and subsequently subjected to a surface postcrosslinking step, **in that** 0.01-25% by weight of ionic surface postcrosslinkers, based on the polymer powder, is added in the form of a 0.01-80% by weight aqueous solution and **in that** the surface postcrosslinking is carried out at temperatures of 40°C to 250°C for a period of 5 minutes to 6 hours.

9. Process according to Claim 8, **characterized in that** the mixing of polycarboxypolysaccharide and water is carried out in a continuous mixer.

10. Process according to Claim 8, **characterized in that** the aqueous solution of the covalent surface postcrosslinker further includes a crosslinking catalyst.

11. Structure for absorbing body fluids, comprising an addition polymer according to any of Claims 1-3 and 5-8.

## Revendications

1. Polymère pulvérulent, post-réticulé en surface, absorbant l'eau, les fluides aqueux ou séreux et le sang, pouvant être obtenu par une réticulation de surface d'au moins un polysaccharide partiellement neutralisé, contenant des groupes carboxyle, **caractérisé en ce que** le polycarboxypolysaccharide est pré-gonflé en milieu aqueux avant la réticulation de surface sous forme non réticulée et de nouveau séché, **en ce que** des agents de réticulation ioniques sont utilisés pour la post-réticulation de surface, **en ce que** les agents de réticulation de surface ioniques sont des sels de cations au moins bivalents, **en ce que** le cation polyvalent est formé par Al³⁺, la poudre polymère présentant un indice de réticulation de surface (SCI) supérieur à 40, le SCI étant calculé à partir de la quantité d'Al³⁺ ajoutée lors de la réticulation de surface, par rapport à la résine superabsorbante (= C_{SAP}) et de la concentration en Al³⁺ des particules fines (= C_{F}) déterminée après sollicitation mécanique selon SCI = (C_{F} - C_{SAP}) * 100, C_{F} et C_{SAP} étant utilisés en % d'Al³⁺.

2. Poudre polymère selon la revendication 1, **caractérisée en ce que** l'agent de réticulation de surface est présent en une quantité de 0,01 à 25 % en poids, par rapport au polycarboxypolysaccharide.

3. Poudre polymère selon la revendication 1, **caractérisée en ce que** l'agent de réticulation de surface est utilisé en une quantité de 0,2 à 1,0 % en poids, par rapport au polycarboxypolysaccharide.

4. Poudre polymère selon les revendications 1 à 3, **caractérisée en ce qu'**elle présente à une rétention supérieure ou égale à 20 g/g une absorption sous pression (AAP_{0,7}) déterminée selon la méthode Edena n° 442.1-99 selon la description d'au moins 11 g/g.

5. Poudre polymère selon les revendications 1 à 3, **caractérisée en ce qu'**elle présente à une rétention supérieure ou égale à 25 g/g une absorption sous pression (AAP_{0,7}) d'au moins 11 g/g.

6. Poudre polymère selon les revendications 1 à 3, **caractérisée en ce qu'**elle présente après un vieillissement de 200 jours en conditions normales au moins 80 % de l'absorption sous pression (AAP_{0,7}) en comparaison de la valeur initiale.

7. Poudre polymère selon les revendications 1 à 3, **caractérisée en ce qu'**elle forme après sollicitation mécanique par broyage par des rouleaux pendant 6 minutes moins de 5 % en poids de fraction de fines ayant une taille de particule inférieure à 150 µm.

8. Procédé de fabrication de polymères absorbants selon l'une quelconque des revendications 1 à 3, par réticulation de la surface d'un polycarboxypolysaccharide avec un agent de réticulation de surface, **caractérisé en ce qu'**un hydrogel est formé à partir d'un polycarboxypolysaccharide non réticulé avec de l'eau, l'hydrogel est broyé mécaniquement et séché, un broyage et une classification de l'hydrogel séché ont lieu avec formation d'une poudre polymère, et **en ce que** les particules de la poudre polymère sont revêtues avec une solution d'un agent de réticulation, puis soumises à une post-réticulation de surface, **en ce que** 0,01 à 25 % en poids d'agents de post-réticulation ioniques, par rapport à la poudre polymère, sous la forme d'une solution aqueuse à 0,01 à 80 % en poids, sont ajoutés, et **en ce que** la post-réticulation de surface est réalisée à des températures de 40 °C à 250 °C pendant une durée de 5 minutes à 6 heures.

9. Procédé selon la revendication 8, **caractérisé en ce que** le mélange du polycarboxypolysaccharide et de l'eau est réalisé dans un mélangeur continu.

10. Procédé selon la revendication 8, **caractérisé en ce que** la solution aqueuse de l'agent de post-réticulation covalent contient en outre un catalyseur de réticulation.

11. Construction destinée à l'absorption de fluides corporels, contenant un polymère selon l'une quelconque des revendications 1 à 3 et 5 à 8.
